(19) Europäisches Patentamt
European Patent Office
Office européen des brevets

(11) Veröffentlichungsnummer : **0 012 361**
**B1**

(12) # EUROPÄISCHE PATENTSCHRIFT

(45) Veröffentlichungstag der Patentschrift : **22.09.82**

(21) Anmeldenummer : **79104950.5**

(22) Anmeldetag : **05.12.79**

(51) Int. Cl.$^3$ : **C 07 D403/04**, C 07 D239/48, C 07 D405/12, C 07 D405/14, A 61 K 31/505

(54) **Amino-pyrimidin-carbanilide, Verfahren zu ihrer Herstellung, diese Verbindungen enthaltende Arzneimittel.**

(30) Priorität : **09.12.78 DE 2853220**

(43) Veröffentlichungstag der Anmeldung : **25.06.80 (Patentblatt 80/13)**

(45) Bekanntmachung des Hinweises auf die Patenterteilung : **22.09.82 Patentblatt 82/38**

(84) Benannte Vertragsstaaten : **AT BE CH DE FR GB IT NL SE**

(56) Entgegenhaltungen :
**JOURNAL OF MEDICINAL CHEMISTRY, Band 15, Nr. 2, 1972, Washington, US, E.L. STOGRYN et al. : « Synthesis of trimethoprim variations. Replacement of $CH_2$ by polar groupings », Seite 200.**

(73) Patentinhaber : **HOECHST AKTIENGESELLSCHAFT**
**Postfach 80 03 20**
**D-6230 Frankfurt/Main 80 (DE)**

(72) Erfinder : **Kampe, Klaus-Dieter, Dr.**
**Am Rehsteig 1**
**D-6232 Bad Soden am Taunus (DE)**
Erfinder : **Granzer, Ernold, Dr.**
**Falkensteiner Strasse 24**
**D-6233 Kelkheim (Taunus) (DE)**

**EP 0 012 361 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# Amino-pyrimidin-carbanilide, Verfahren zu ihrer Herstellung, diese Verbindungen enthaltende Arzneimittel

Die Erfindung betrifft neue 4-Amino-2-ureido-pyrimidin-5-carbonsäure-anilide einschließlich ihre Salze, Verfahren zu ihrer Herstellung, diese Verbindungen enthaltende Arzneimittel und ihre Verwendung insbesondere bei der Behandlung von Lipidstoffwechselstörungen und Adipositas.

Nahezu alle im Handel befindlichen Anorektika sind Derivate des β-Phenylethylamins. Genannt seien hier als Beispiele Phenmetrazin, Amfepramon, Norpseudoephedrin, Fenfluramin, und Fenproporex. Diese Präparate haben den Nachteil, daß sie mehr oder weniger ausgeprägte Nebenwirkungen auf das Zentralnervensystem und/oder den Blutkreislauf ausüben [vgl. D. Craddock, Drugs 11, 378 (1976); P. H. Connell, Side Effects of Drugs, Ed. 2, Ed. M. N. G. Dukes, Excerpta Medica, Amsterdam 1978].

Als neueres Präparat mit appetitszügelnder Wirkung ist in jüngster Zeit ein Imidazo [2.1-a] isoindol-Abkömmling unter der Bezeichnung Mazindol bekannt geworden [J. H. Gogerty u.a. Arch. int. Pharmacodyn. 214, 285 (1975)]. Obgleich es sich bei dieser Verbindung nicht um ein Derivat des β-Phenylethylamins handelt, ist dieses Anorektikum ebenfalls nicht frei von Nebenwirkungen auf das Zentralnervensystem.

Das pharmakologische Wirkungsprofil gleicht in mehrfacher Hinsicht dem von Amphetamin und verwandten Substanzen; Mazindol wirkt wie Amphetamin durch eine Erhöhung des Dopaminumsatzes zentralstimulierend. Der bei chronischer Gabe gelegentlich schnell eintretende Wirkungsverlust ist möglicherweise auf eine durch Mazindol induzierte Hyperinsulinämie zurückzuführen. Bei der Behandlung mit Mazindol traten die typischen Nebenwirkungen zentralstimulierender Appetitzügler auf, wie Schlaflosigkeit, Schwindel, Obstipation, Nervosität, Tachykardie und starkes Schwitzen, Zusätzlich kommt Mundtrockenheit sowie eine stark ausgeprägte antidepressive Wirkung hinzu. Bei amphetaminabhängigen Patienten wirkt Mazindol amphetaminähnlicher als Fenfluramin. [vgl. Arznei-Telegramm 12/76, S. 92; M. Babbini, M. Gaiardi und M. Bartoletti, Pharmacology 15, 46 (1977); A. Kornhaber, Psychosomatics 14, 162 (1973)].

Der Erfindung liegt die Aufgabe zugrunde, ein appetitzügelndes Präparat zur Verfügung zu stellen, das diese Nebenwirkungen nicht zeigt und das insbesondere zu keiner unerwünschten Beeinflussung des Herz-Kreislauf- und Zentralnervensystems führt.

Es wurde nun gefunden, daß

4-Amino-2-ureido-(bzw.-thioureido)-pyrimidin-5-carbonsäure-anilide der allgemeinen Formel I

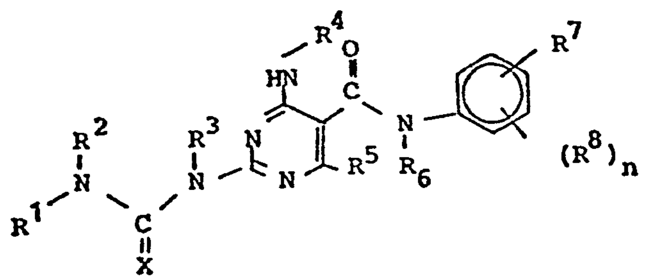

(I)

in der

$R^1$ und $R^3$, die gleich oder verschieden sind, Wasserstoff, eine $(C_1-C_3)$-Alkylgruppe oder einen Phenylrest,

$R^2$ Wasserstoff, eine $(C_1-C_{10})$-Alkylgruppe, eine $(C_3-C_4)$-Alkenylgruppe, eine $(C_4-C_8)$-Cycloalkylgruppe, eine Benzylgruppe oder eine Phenylgruppe, worin die Phenylreste zusätzlich durch 1-2 $(C_1-C_3)$-Alkylgruppen, 1-2 Halogenatome, 1-2 $(C_1-C_4)$-Alkoxigruppen, eine Trifluormethylgruppe, eine Methoxi- oder Ethoxicarbonylgruppe und/oder eine Methylendioxigruppe substituiert sein können,

$R^4$ Wasserstoff oder eine $(C_1-C_8)$-Acylgruppe,

$R^5$ Wasserstoff,

$R_6$ Wasserstoff, eine $(C_1-C_3)$-Alkylgruppe, eine $(C_3-C_4)$-Alkenylgruppe, eine, gegebenenfalls durch 1 oder 2 Chloratome oder 1 oder 2 Methoxigruppen im Phenylrest substituierte, Benzylgruppe oder einen, gegebenenfalls durch 1 Methylgruppe und/oder 1 Chloratom substituierten, Phenylrest,

$R^7$ Wasserstoff, eine $(C_1-C_3)$-Alkylgruppe, eine $(C_3-C_4)$-Alkenylgruppe, eine $(C_5-C_6)$-Cycloalkylgruppe, eine Phenylgruppe, ein Halogenatom, eine Trifluormethyl-, $(C_1-C_4)$-Alkylthio-, $(C_1-C_2)$-Alkoxicarbonyl-, Cyano-, Acetamino-, Amino-, Nitro-, Carboxyl- oder $(C_1-C_4)$-Alkoxigruppe oder einen Rest

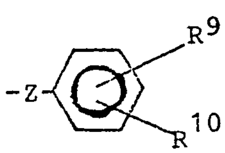

worin

Z ein Sauerstoff- oder Schwefelatom oder eine $-\overset{O}{\overset{\|}{C}}-$, $-CH_2-$ oder $-CH_2CH_2-$Gruppe und

$R^9$ und $R^{10}$, die gleich oder verschieden sind, ein Wasserstoff-, Fluor-, Chlor- oder Bromatom, eine $(C_1$-$C_3)$-Alkylgruppe, eine $(C_1$-$C_2)$-Alkoxigruppe, eine Acetamino- oder eine $(C_1$-$C_2)$-Alkoxicarbonylgruppe oder eine Carboxylgruppe bedeuten,

$R^8$ ein Fluor-, Chlor- oder Bromatom oder eine Methyl-, $(C_1$-$C_2)$-Alkoxi- oder Trifluormethylgruppe,

X ein Sauerstoff- oder Schwefelatom und

n = 0, 1, 2 oder 3 bedeuten, wobei die Reste

$R^1$ und $R^2$ zusammen auch einen verzweigten oder unverzweigten Alkylenrest mit 3-6 C-Atomen oder den $(-CH_2CH_2OCH_2CH_2-)$-Rest und

$R^2$ und $R^3$ zusammen einen verzweigten oder unverzweigten Alkylenrest mit 2-8 C-Atomen, der zusätzlich durch eine $(C_2$-$C_4)$-Alkenylgruppe oder einen, gegebenenfalls mit einem Chlor- oder Bromatom oder einer Methyl-, Ethyl-, Methoxi- oder Ethoxigruppe substituierten, Phenylrest substituiert sein kann, oder den (-CH=CH-)-Rest bedeuten, und wobei im Fall n = 1, die Reste $R^7$ und $R^8$ zusammen auch eine Methylen- oder Ethylendioxigruppe bedeuten können,

sowie deren physiologisch verträglichen Säureadditionssalze wertvolle pharmakologische Eigenschaften besitzen. Insbesondere sind sie anorektisch wirksam, d.h. durch ihre appetitzügelnde Wirkung vermögen sie Übergewicht, Fettleibigkeit etc. zu verhindern. Außerdem sind sie zur Behandlung von Lipidstoffwechselstörungen geeignet.

Die Erfindung betrifft daher 4-Amino-2-ureido- (bzw. -thioureido)-pyrimidin-5-carbonsäure-anilide der allgemeinen Formel I sowie deren physiologisch verträgliche Säureadditionssalze.

Bevorzugt sind diejenigen Verbindungen der allgemeinen Formel I mit folgenden Bedeutungen der Substituenten :

$R^1$ Wasserstoff, Methyl oder Ethyl,

$R^2$ Wasserstoff, eine $(C_1$-$C_8)$-Alkylgruppe, eine $(C_3$-$C_4)$-Alkenylgruppe, eine $(C_5$-$C_6)$-Cycloalkylgruppe, eine Benzyl- oder Phenylgruppe, die gegebenenfalls im Phenylrest zusätzlich durch eine Methylgruppe, 1 oder 2 Chloratome, 1 oder 2 Methoxigruppen, eine Ethoxi- oder Trifluormethyl- und/oder eine Methylendioxigruppe substituiert sein können,

$R^3$ Wasserstoff oder zusammen mit $R^2$ wie unten angegeben,

$R^4$ Wasserstoff oder Acetyl,

$R^6$ Wasserstoff, eine $(C_1$-$C_3)$-Alkylgruppe, eine Allylgruppe oder eine (gegebenenfalls durch ein Chloratom substituierte) Benzylgruppe,

$R^7$ eine $(C_1$-$C_3)$-Alkylgruppe, eine Phenylgruppe, eine Halogenatom, eine $CF_3$-, $(C_1$-$C_4)$-Alkylthio-, Nitro-, $(C_1$-$C_2)$-Alkoxicarbonyl- oder $(C_1$-$C_4)$-Alkoxigruppe oder einen Rest

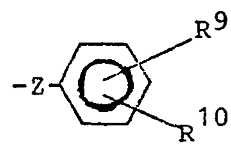

worin Z ein 0- oder S-Atom und $R^9$ und $R^{10}$ gleich oder verschieden sind und ein Wasserstoff-, F-, Cl- oder Br-Atom, eine Methyl- oder Methoxygruppe bedeuten,

$R^8$ Methyl, Fluor, Chlor, Brom, eine $(C_1$-$C_2)$-Alkoxi- oder Trifluormethylgruppe wobei

n = 0, 1 oder 2 ist, und

X ein Sauerstoffatom und die Reste

$R^1$ und $R^2$ zusammen auch einen Tetra- oder Pentamethylen- oder den $(-CH_2CH_2OCH_2CH_2-)$-Rest oder die Reste

$R^2$ und $R^3$ zusammen auch eine verzweigte oder unverzweigte Alkylenkette mit 2-6 C-Atomen, die zusätzlich durch eine Vinyl- oder Phenylgruppe substituiert sein kann ; und wobei die eigentliche Alkylenkette, die mit der Ureidogruppe zusammen den Ring bildet, 2 C-Atome enthält, und im Fall n = 1 die Reste

$R^7$ und $R^8$ zusammen auch eine 3,4-ständige Methylendioxigruppe.

Besonders bevorzugt sind Verbindungen der allgemeinen Formel I mit folgenden Bedeutungen der Substituenten :

$R^2$ eine $(C_1$-$C_8)$-Alkylgruppe, eine Allyl-, Cyclohexyl-, eine, gegebenenfalls durch ein Chloratom substituierte, Benzylgruppe oder eine gegebenenfalls durch 1 oder 2 Chloratome, eine Methyl-, Trifluormethyl- oder Methoxigruppe substituierter Phenylrest

$R^3$ Wasserstoff oder zusammen mit $R^2$ wie unten angegeben,

$R^1$ und $R^4$ Wasserstoff,

$R^6$ Wasserstoff, einen Methyl-, Ethyl-, Allyl- oder Benzylrest

$R^7$ eine $(C_1\text{-}C_3)$-Alkylgruppe, eine Phenylgruppe, ein Fluor-, Chlor- oder Bromatom, eine $CF_3$- oder $(C_1\text{-}C_2)$-Alkoxigruppe oder einen Rest

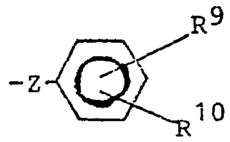

worin Z ein O- oder S-Atom $R^9$ und $R^{10}$ gleich oder verschieden sind und ein Wasserstoff- oder Chloratom, eine Methyl- oder Methoxigruppe bedeuten,

$R^8$ Methyl, Fluor, Chlor, eine $(C_1\text{-}C_2)$-Alkoxi- oder Trifluormethylgruppe und

X eine Sauerstoffatom und

n = 0, 1 oder 2,

$R^2$ und $R^3$ zusammen auch eine verzweigte oder unverzweigte Alkylerkette mit 2-6 C-Atomen, die zusätzlich durch eine Vinyl- oder Phenylgruppe substituiert sein kann, und wobei die eigentliche Alkylenkette, die mit der Ureidogruppe zusammen den Ring bildet, aus 2 C-Atomen besteht, und außerdem

$R^7$ und $R^8$ im Fall n = 1 zusammen auch eine 3,4-ständige Methylendioxigruppe.

Falls der Substituent $R^8$ mehrmals vorkommt, so sind seine Bedeutungen gleich oder verschieden.

Als Salze der erfindungsgemäßen Verbindungen der allgemeinen Formel I kommen solche mit physiologisch unbedenklichen anorganischen und organischen Säuren, wie beispielsweise Chlor-, Brom- oder Jodwasserstoff-, Schwefel-, Phosphor-, Salpeter-, Benzolsulfon-, Toluolsulfon-, Sulfamin-, Methylschwefel-, Essig-, Propion-, Oel-, Palmitin-, Stearin, Malon-, Malein-, Bernstein-, Glutar-, Aepfel-, Wein-, Zitronen-, Fumar-, Milch-, Glykol-, Benztrauben-, Benzoe-, Toluyl-, Glutamin-, Furancarbon-, Salicyl- oder Mandelsäure in Betracht. Bevorzugt werden Salze mit physiologisch verträglichen anorganischen Säuren oder stark bis mittelstark sauren Derivaten solcher Säuren.

Die Erfindung betrifft weiterhin ein Verfahren zur Herstellung von 4-Amino-2-ureido (bzw. -thioureido)-pyrimidin-5-carbonsäureaniliden der allgemeinen Formel I, das dadurch gekennzeichnet ist, daß man

a) ein Isocyanat der allgemeinen Formel IIa

$$R^2\text{—}N{=}C{=}O \qquad \text{(IIa)}$$

in der $R^2$ außer Wasserstoff die oben angegebenen Bedeutungen hat, mit Guanidin umsetzt und die dabei gebildete Reaktionsmischung anschließend weiter mit einer Verbindung der allgemeinen Formel III

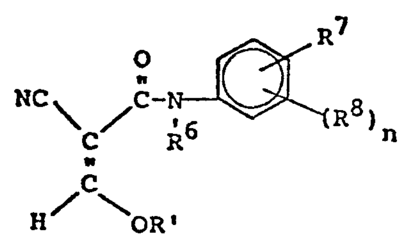

(III)

in der $R^6$, $R^7$ und $R^8$ sowie n die oben bei der allgemeinen Formel I angegebenen Bedeutungen haben und R' eine Methyl- oder Ethylgruppe bedeutet, umsetzt oder

b) Verbindungen der allgemeinen Formel IV

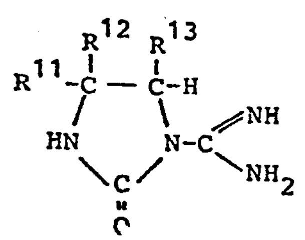

(IV)

in der $R^{11}$ Wasserstoff, $(C_1\text{-}C_3)$-Alkyl-, $(C_2\text{-}C_4)$-Alkenyl oder, gegebenenfalls durch ein Chlor- oder Bromatom oder eine Methyl-, Ethyl-, Methoxi- oder Ethoxigruppe substituiertes Phenyl, und $R^{12}$ und $R^{13}$, die gleich oder verschieden sein können, jeweils Wasserstoff oder eine $(C_1\text{-}C_3)$-Alkylgruppe bedeuten, oder deren Säureadditionssalze und für den Fall, daß Salze von Verbindungen der allgemeinen Formel IV, verwendet werden, unter Zusatz einer basischen Verbindung mit einer Verbindung der allgemeinen

Formel III, in der $R^6$-$R^8$, R' und n die oben angegebene Bedeutung haben, unter Bildung einer Verbindung der allgemeinen Formel I, in der X = 0 bedeutet und $R^1$, $R^4$ und $R^5$ Wasserstoff, und $R^2$ und $R^3$ zusammen eine

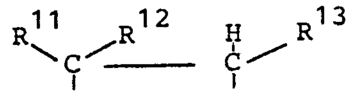

Gruppierung bedeuten, in der $R^{11}$-$R^{13}$ die obige Bedeutung haben, und $R^6$, $R^7$ und $R^8$ sowie n die oben genannte Bedeutung haben, umsetzt oder

c) ein Isocyanat der allgemeinen Formel IIa, oder ein Isothiocyanat der allgemeinen Formel IIb

$$R^2{-}N = C = S \qquad \text{(IIb)}$$

in der $R^2$ außer Wasserstoff die oben angegebene Bedeutung hat, mit einer Verbindung der allgemeinen Formel V

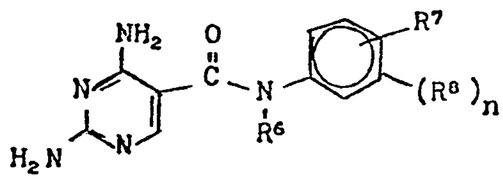

(V)

in der $R^6$, $R^7$ und $R^8$ sowie n die oben bei der allgemeinen Formel I angegebenen Bedeutungen haben, umsetzt,

d) ein Chlorameisensäureamid bzw. ein Chlorthioameisensäureamid der allgemeinen Formel VI

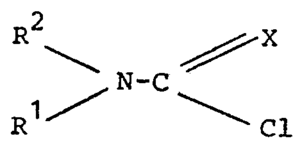

(VI)

in der $R^1$ eine ($C_1$-$C_3$)-Alkylgruppe oder einen Phenylrest, $R^2$ eine ($C_1$-$C_8$)-Alkylgruppe, eine ($C_3$-$C_4$)-Alkenylgruppe, eine ($C_5$-$C_6$)-Cycloalkylgruppe, eine Benzyl- oder Phenylgruppe, die gegebenenfalls im Phenylrest zusätzlich durch eine Methylgruppe, 1 oder 2 Chloratome, 1 oder 2 Methoxigruppen oder eine Methylendioxigruppe substituiert sein können, oder

$R^1$ und $R^2$ zusammen einen verzweigten oder unverzweigten Alkylenrest mit 3-6 C-Atomen oder den ($-CH_2CH_2OCH_2CH_2-$)-Rest und

X ein Sauerstoff- oder Schwefelatom bedeuten mit einer Verbindung der allgemeinen Formel V, in der $R^6$, $R^7$ und $R^8$ sowie n die oben bei der allgemeinen Formel I angegebene Bedeutung haben, umsetzt, oder

e) 4-Amino-2-ureido- (bzw.-2-thioureido)-pyrimidin-5-carbonsäure-anilide der allgemeinen Formel I, in der $R^4$ Wasserstoff bedeutet und $R^1$, $R^2$, $R^3$, $R^5$, $R^6$, $R^7$, $R^8$, X und n die oben bei der allgemeinen Formel I angegebenen Bedeutungen haben, mit einem Acylierungsmittel der allgemeinen Formel VII

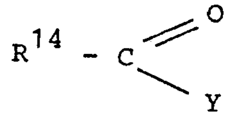

(VII)

in der

$R^{14}$ eine ($C_1$-$C_7$)-Alkylgruppe, einen Cyclohexyl-, einen Benzyl- oder einen, gegebenenfalls durch eine Methylgruppe substituierten Phenylrest und

Y ein Chlor- oder Bromatom oder die Gruppe $R^{14}{-}\overset{\overset{O}{\|}}{C}{-}O{-}$ bedeutet, umsetzt, und gegebenenfalls die nach Weg a)-e) erhaltenen Verbindungen der allgemeinen Formel I mit organischen oder anorganischen Säuren in ihre physiologisch verträglichen Säureadditionssalze überführt.

Die unter a) bis e) genannten Reaktionen können in Anoder Abwesenheit eines Lösungs- bzw. Verdünnungsmittels durchgeführt werden.

Die unter a) bezeichnete Verfahrensweise wird vorteilhaft so ausgeführt, daß man ein Isocyanat der allgemeinen Formel II a zweckmäßig unter Mitverwendung eines Lösungsmittels in Mengen von 1,0-1,3 Aequivalenten bei einer Temperatur zwischen − 50 und + 60 °C insbesondere zwischen − 20 und 35 °C mit 1 Aequivalent Guanidin umsetzt und die dabei entstandene Reaktionsmischung, ohne Isolierung des gebildeten Guanylharnstoffs der allgemeinen Formel VIII, gegebenenfalls unter Verwendung weiterer zusätzlicher, gegebenenfalls anderer, Lösungsmittel, weiter mit einer Verbindung der allgemeinen Formel III bei einer Temperatur zwischen − 20 und + 250 °C — vorzugsweise zwischen + 15 und + 140 °C — umsetzt. Die dabei entstehenden Verbindungen der allgemeinen Formel I ($R^1$, $R^3$, $R^4$, $R^5$ = H ; $R^2$, $R^6$, $R^7$, $R^8$ und n mit obiger Bedeutung) fallen meist in kristalliner Form an oder werden nach Verdampfen der Lösungsmittel nach üblichen Methoden in kristalliner Form erhalten und isoliert. Bevorzugt werden für diese Verfahrensweise Verbindungen der allgemeinen Formel III verwendet, bei denen $R^6$ Wasserstoff bedeutet und $R^7$, $R^8$ und R′ sowie n die oben angegebenen Bedeutungen haben. Die unter a) bezeichnete Verfahrensweise kann durch das nachstehende Formelschema veranschaulicht werden.

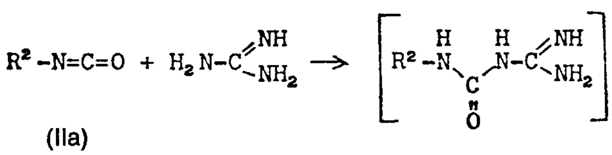

(IIa) (VIII)

(VIII) +

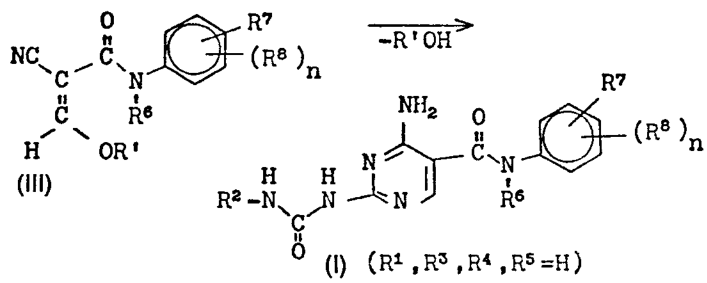


(III)

(I) ($R^1$, $R^3$, $R^4$, $R^5$=H)

Als Lösungsmittel für diese Reaktion können beispielsweise niedere Alkanole, wie Methanol, Ethanol, Propanol, Isopropanol, Butanol, Isobutanol, tert.-Butanol und/oder Butandiol, Ether, Tetrahydrofuran, 1,2-Dimethoxiethan, Dioxan, Acetonitril, Dimethylformamid, Dimethylacetamid, Sulfolan, Sulfolen, Chloroform, Hexamethylphosphorsäuretriamid, Dimethylsulfoxid, Diglykoldimethylether, Methylenchlorid oder Ethylacetat verwendet werden. Geeignete Isocyanate sind neben den in den Beispielen genannten beispielsweise Methyl-, Ethyl-, Isopropyl-, Isobutyl-, Pentyl-, Hexyl-, 2-Ethyl-hexyl-, Decyl-, Cyclopentyl-, Cycloheptyl-, Cyclooctyl-, Allyl-, Methallyl-, Phenyl-, 4-Methoxi-phenyl-, 3,4-Dimethoxiphenyl-, 4-Ethoxi-phenyl-, 4-Butoxi-phenyl-, o-, m- oder p-Toluyl-, 4-Isopropyl-phenyl-, 4-Fluor-phenyl-, 3-Ethoxicarbonyl-phenyl- oder 3,4-Methylendioxi-phenyl-, Benzyl-, 4-Chlorbenzyl-, 4-Methoxibenzyl-, 2-Phenyl-ethyl-oder 2-(3-Methoxiphenyl)-ethyl-isocyanat.

Die als Ausgangsstoffe zu verwendenden Isocyanate II a sind größtenteils bekannte Verbindungen oder können nach bekannten Methoden hergestellt werden [vgl. dazu z.B. Liebigs Ann. d. Chem. *562,* 75-136 (1949) und Houben-Weyl, Methoden, d. Org. Chemie 4. Aufl. Bd. VIII, Teil 3 S. 120, Stuttgart 1952].

Die 3-Alkoxi-2-cyano-acrylsäure-anilide der allgemeinen Formel III können aus entsprechenden Cyanacetaniliden durch Umsetzung mit Orthoameisensäureestern in Gegenwart von Acetanhydrid und vorteilhaft unter Zusatz von Lewissäuren als Katalysatoren hergestellt werden (vgl. dazu DE-OS 25 55 789).

Die unter b) bezeichnete Verfahrensweise wird vorteilhaft so ausgeführt, daß man ein Amidinoimidazolidinon der allgemeinen Formel IV bei einer Temperatur zwischen − 30 und + 250 °C — vorzugsweise zwischen + 15 und + 140 °C — zweckmäßig in Gegenwart eines Lösungsmittels mit einer Verbindung der allgemeinen Formel III umsetzt. Für diese Reaktion können die Verbindungen der allgemeinen Formel IV als freie Basen oder in Form ihrer Säureadditionssalze verwendet werden. Werden Salze verwendet, dann setzt man, vorteilhaft mindestens 1 Aequivalent oder mehr, einer basischen Verbindung zu. Bevorzugt wird die Umsetzung der Salze von Verbindungen der allgemeinen Formel IV mit basischen Verbindungen bei einer Temperatur zwischen − 30 und + 50° vor der Zugabe des anderen Reaktionspartners, den Verbindungen der allgemeinen Formel III vorgenommen. Nach einer Reaktionszeit von einigen Minuten bis zu mehreren Stunden kann dann anschließend im gleichen Reaktionsgefäß, ohne Isolierung der Amidino-imidazolidinone der allgemeinen Formel IV die Umsetzung mit den Verbindungen der allgemeinen Formel III durchgeführt werden.

Als basische Verbindungen können beispielsweise Alkali- oder Erdalkali-alkanolate von niederen Alkanolen, Alkali- oder Erdalkali-hydroxide, -carbonate oder hydrogencarbonate, Natriumhydrid oder tertiäre Amine wie Triethylamin oder N,N-Dimethylanilin verwendet werden.

Als Lösungsmittel können beispielsweise diejenigen verwendet werden, die bei der unter a) bezeichneten Verfahrensweise genannt worden sind.

Die 1-Amidino-imidazolidin-2-one der allgemeinen Formel IV, wenden vorzugsweise nach dem in der DE-OS 28 53 221 beschriebenen Verfahren hergestellt. Das dort beschriebene Verfahren ist dadurch gekennzeichnet, daß man 2-Halogenalkyl-isocyanate der Formel

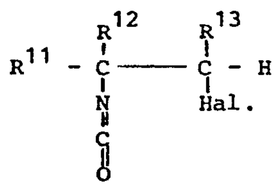

worin Hal. ein Chlor-, Brom- oder Jodatom bedeutet und $R^{11}$, $R^{12}$ und $R^{13}$ die zur allgemeinen Formel IV angegebenen Bedeutungen haben, gegebenenfalls in Gegenwart eines Lösungs- bzw. Verdünnungsmittels mit Guanidin umsetzt.

Bevorzugt werden soche Verbindungen der allgemeinen Formel IV, bei denen entweder die Reste $R^{11}$, $R^{12}$ und $R^{13}$ Wasserstoff oder $R^{11}$ eine ($C_1$-$C_3$)-Alkyl- oder Alkenylgruppe oder Phenyl und $R^{12}$ eine Methyl- oder Ethylgruppe oder Wasserstoff und $R^{13}$ eine Methylgruppe oder Wasserstoff bedeuten, wobei jedoch $R^{12}$ Wasserstoff darstellt, wenn $R^{13}$ eine Methylgruppe bedeutet.

Für die Durchführung der Reaktion ist es nicht nötig, die Verbindungen der allgemeinen Formel IV oder ihre Säureadditionssalze in reiner, kristalliner Form zu verwenden. Statt dessen können die bei der Herstellung dieser Verbindungen mitunter ölig anfallenden Rohprodukte eingesetzt werden.

Die 50 gebildeten Verbindungen der allgemeinen Formel I fallen häufig als praktisch reine, kristalline Stoffe aus dem Reaktionsmedium aus, oder sie werden nach Verdampfen der Lösungsmittel mit üblichen Methoden isoliert und im Bedarfsfall durch Umkristallisation oder durch Chromatographie gereinigt.

Die unter c) bezeichnete Verfahrensweise wird vorteilhaft so ausgeführt, daß man ein Isocyanat II a oder Isothiocyanat IIb unter Mitverwendung eines Lösungsmittels und einer, keine N-H-Bindungen enthaltenden basischen Stickstoffverbindung bei einer Temperatur zwischen 0 und 250 °C — vorzugsweise zwischen 40 und 150 °C — mit einer Verbindung der allgemeinen Formel V umsetzt. Bevorzugt wird diese Umsetzung mit Isocyanaten II a ausgeführt.

Als Lösungsmittel eignen sich beispielsweise außer den Alkoholen diejenigen, die bei unter a) bezeichneten Verfahrensweise genannt worden sind. Bevorzugt werden polare aprotische Lösungsmittel wie Acetonitril, N,N-Dimethylformamid, Dimethylsulfoxid, Hexamethylphosphorsäuretriamid, Dioxan, Dimethoxiethan, Sulfolan, Sulfolen, Diglykoldimethylether (Diglyme) oder Pyridin.

Es können auch Gemische der aufgeführten Lösungsmittel angewendet werden, sowie auch Mischungen der aufgeführten mit anderen Lösungsmitteln verwendet werden können.

Die Verbindungen der allgemeinen Formel V sowie die daraus erfindungsgemäß gebildeten Verbindungen der allgemeinen Formel I können im jeweiligen Lösungs- bzw. Verdünnungsmittel suspendiert oder gelöst vorliegen. Der Anteil der gelösten Stoffe hängt stark vom Lösevermögen der jeweiligen Lösungsmittel ab und steigt im allgemeinen mit deren Polarität.

Unter den vorteilhaft als Katalysatoren mitzuverwendenden basischen Stickstoffverbindungen, die keine NH-Gruppierung enthalten dürfen, sind Stickstoffbasen wie tertiäre Amine, vollständig alkylsubstituierte Amidine, darunter auch bicyclische Vertreter und/oder heteroaromatische cyclische Stickstoffverbindungen zu verstehen. Geeignete tertiäre Amine sind beispielsweise Triethyl- oder Tributylamin, Diethyl-cyclohexyl-amin, N-Ethyl-piperidin, N,N-Dimethyl- oder-Diethylanilin oder Diaza [2,2,2] bicyclooctan.

Geeignete Amidine sind beispielsweise Diazabicycloalkane wie z.B. die Verbindung IX

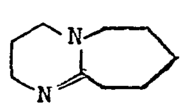
(IX)

und geeignete heteroaromatische cyclische Stickstoffbasen sind zum Beispiel Pyridin, Picolin, Chinolin, Chinaldin und/oder N-Methyl-imidazol.

Diese vorteilhaft als Katalysatoren mitverwendeten basischen Stickstoffverbindungen werden zweckmäßig in Mengen von 0,01-2,00, vorzugsweise 0,10-1,10 Mol, bezogen auf 1 Mol einer Verbindung der allgemeinen Formel IIa bzw. IIb angewandt.

Die Reaktion zwischen den Verbindungen der allgemeinen Formel IIa bzw. b und der allgemeinen Formel V kann aber auch ohne Zusatz einer basischen Stickstoffverbindung ausgeführt werden.

Die Mengenverhältnisse, in denen die Isocyanate IIa bzw. Isothiocyanate IIb mit Verbindungen der allgemeinen Formel V zur Reaktion gebracht werden, können erfindungsgemäß stark schwanken und können zwischen 0,30 bis 3,00 Mol oder mehr einer Verbindung der allgemeinen Formel IIa bzw. b pro Mol einer Verbindung der allgemeinen Formel V liegen. Bevorzugt werden 0,90 bis 1,60 Mol einer Verbindung der allgemeinen Formel II a bzw. b pro Mol einer Verbindung der allgemeinen Formel V angewendet.

Die 2,4-Diamino-pyrimidin-5-carbanilide der allgemeinen Formel V sind neue Verbindungen. Sie sind wertvolle Zwischenprodukte und dienen insbesondere zur Herstellung von Verbindungen der allgemeinen Formel I. Sie können hergestellt werden durch Umsetzung von Guanidin oder einem Guanidiniumsalz mit einer Verbindung der allgemeinen Formel III. Diese Umsetzung wird analog der oben unter a) oder b) bezeichneten Verfahrensweise ausgeführt. Die für diese Verfahrensweise oben näher erläuterten Reaktionsbedingungen gelten somit ebenso für die Herstellung der Verbindungen der allgemeinen Formel V. Werden beispielsweise Guanidiniumsalze verwendet, so arbeitet man zweckmäßig unter Zusatz einer basischen Verbindung wie oben unter a) bzw. b) erläutert wurde. Die Bildung der Verbindungen der allgemeinen Formel V wird durch nachstehendes Formelschema veranschaulicht.

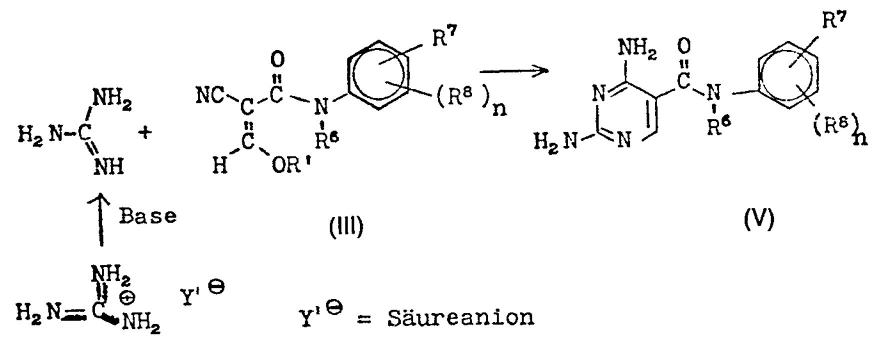


(III) (V)

$Y'^{\ominus}$ = Säureanion

Die für die allgemeine Formel III im Bezug auf die E-/Z-Isomerie gewählte Schreibweise ist willkürlich. Sie soll beide Formen einschließen. Für die vorstehend beschriebene Umsetzung und die unter a) und b) bezeichneten Verfahrensweisen können die Verbindungen der allgemeinen Formel III sowohl als reine E- oder Z-Form als auch als E- und Z-Mischungen eingesetzt werden. In jedem Fall bilden sich unter Ringschluß die jeweiligen erfindungsgemäßen Pyrimidin-Derivate.

Die unter d) bezeichnete Verfahrensweise wird vorteilhaft so ausgeführt, daß man ein Chlorameisensäureamid oder thioamid der allgemeinen Formel VI, in der $R^1$, $R^2$ und X die oben angegebene Bedeutung haben, zweckmäßig unter Mitverwendung eines gegenüber diesen Verbindungen der allgemeinen Formel VI inerten Lösungs- bzw. Verdünnungsmittels in Mengen von 1,0-1,8 Aequivalenten bei einer Temperatur zwischen 18 und 140 °C — vorzugsweise zwischen 40 und 100 °C — in Gegenwart von zweckmäßig 0,8-1,3 Aequivalenten einer gegenüber den Verbindungen der allgemeinen Formel VI inerten basischen Verbindung auf eine Verbindung der allgemeinen Formel V einwirken läßt. Für diese Umsetzung werden bevorzugt Chlorameisensäureamide der allgemeinen Formel VI, in der X ein Sauerstoffatom bedeutet, verwendet.

Als basische Verbindungen, die vorteilhaft zur Bindung des bei der Reaktion gebildeten Chlorwasserstoffs mitverwendet werden, eignen sich beispielsweise die bei der Erläuterung der unter c) bezeichneten Verfahrensweise auf S. 15 genannten. Die Umsetzung der Verbindungen der allgemeinen Formel VI mit denen der allgemeinen Formel V kann grundsätzlich auch ohne Zusatz einer solchen basischen Verbindung ausgeführt werden.

Als Lösungs- bzw. Verdünnungsmittel sind aprotische, vorzugsweise polare Lösungsmittel geeignet, wie beispielsweise Tetrahydrofuran, Ether, 1,2-Dimethoxyethan, Dioxan, Acetonitril, Sulfolan, Chloroform, Methylenchlorid, 1,2-Dichlorethan, Ethylacetat, Aceton, Dimethylsulfoxid oder Pyridin.

Es können auch Mischungen der aufgeführten Lösungsmittel verwendet werden. sowie auch Mischungen der aufgeführten mit anderen Lösungsmitteln verwendet werden können.

Die Verbindungen der allgemeinen Formel V sowie die daraus durch Einwirkung von Chlorameisensäureamiden bzw. -thioamiden der allgemeinen Formel VI gebildeten Verbindungen der allgemeinen Formel I können im jeweiligen Lösungs- bzw. Verdünnungsmittel suspendiert oder gelöst vorliegen. Der

Anteil der gelösten Stoffe hängt stark vom Lösevermögen der jeweiligen Lösungsmittel und von den Substituenten im Phenylrest der Anilidgruppe der Verbindungen der allgemeinen Formel V bzw. Iab. Im allgemeinen steigt das Lösevermögen im Bezug auf die Verbindungen der allgemeinen Formel V und I mit der Polarität der Lösungsmittel. Weitere Einzelheiten bezüglich der Ausführung der mit d) bezeichneten Verfahrensweise können den Beispielen entnommen werden. Die Chlorameisensäureamide der allgemeinen Formel VI (X = 0) sind zum großen Teil bekannte, oder können nach bekannten Methoden [vgl. J. Chem. Soc. *1947*, 307 und Houben-Weyl, Meth. d. Organ. Chem. Bd. VIII, Teil 3, S. 117 (Stuttgart 1952)] hergestellt werden. Die Synthese der Chlorameisensäurethioamide der allgemeinen Formel VI (X = S) ist ebenfalls in der Fachliteratur beschrieben worden [vgl. Houben-Weyl, Meth. d. Organ. Chem. Bd IX, S. 830 (Stuttgart 1955)].

Das mit e) bezeichnete Verfahren zur Acylierung von Verbindungen der allgemeinen Formel I, in der $R^4$ Wasserstoff bedeutet und die übrigen Reste die oben angegebene Bedeutung haben, wird vorteilhaft so ausgeführt, daß man, gegebenenfalls unter Mitverwendung eines gegenüber dem Acylierungsmittel der allgemeinen Formel VII inerten Lösungsmittels, bei einer Temperatur zwischen −20 und 160 °C — vorzugsweise zwischen 20 und 125 °C — ein Acylierungsmittel der allgemeinen Formel VII auf eine Verbindung der allgemeinen Formel I, in der $R^4$ Wasserstoff bedeutet, einwirken läßt. Werden Säurechloride oder — bromide als Acylierungsmittel verwendet, dann setzt man vorteilhaft — zweckmäßig mindestens I Aequivalent — eine gegenüber Säurechloriden und — bromiden unter den Reaktionsbedingungen inerte basische Verbindung zu und arbeitet vorzugsweise unter Mitverwendung geeigneter Lösungsmittel. Als basische Verbindungen eignen sich die üblicherweise als Säurebindemittel zu verwendenden Basen wie beispielsweise tertiäre Amine, vollständig alkylsubstituierte Amidine (vgl. S. 15) heteroaromatische cyclische Stickstoffbasen wie Pyridin, Picolin, Chinolin, Chinaldin und/oder N-Methylimidazol und/oder Alkali- oder Erdalkalikarbonate und/oder -hydrogenkarbonate.

Die Acylierung mit Säurechloriden oder -bromiden kann grundsätzlich auch ohne Zusatz eines solchen basischen Säurebindemittels ausgeführt werden.

Die Säurechloride oder -bromide werden in mindestens äquivalenten Mengen oder im Ueberschuß angewendet.

Werden Carbonsäureanhydride als Acylierungsmittel verwendet, dann werden diese in mindestens äquivalenten Mengen, vorzugsweise im Ueberschuß, gegebenenfalls unter Mitverwendung geeigneter Lösungsmittel angewandt. Arbeitet man ohne zusätzliche Lösungsmittel, dann setzt man die Carbonsäureanhydride vorteilhaft im Ueberschuß ein, der beispielsweise die 3- bis 30-fache molare Menge, bezogen auf die zu acylierende Verbindung der allgemeinen Formel I ($R^4$ = H), betragen kann.

Als gegebenenfalls mitzuverwendende Lösungsmittel bei der mit e) bezeichneten Verfahrensweise eignen sich alle bekannten, gegenüber den Acylierungsmitteln der allgemeinen Formel VII inerten Lösungsmittel. Vorzugsweise werden polare, aprotische Lösungsmittel wie beispielsweise Acetonitril, Butyronitril, Tetrahydrofuran, 1,2-Dimethoxiethan, Dioxan, Dimethylacetamid, Methylenchlorid, Hexamethylphosphorsäuretriamid, Dimethylsulfoxid, Sulfolan, Chloroform oder Aceton verwendet.

Selbstverständlich können auch Gemische der aufgeführten Lösungsmittel verwendet werden, wie auch Mischungen der aufgeführten Lösungsmittel mit anderen Solventien.

Die Verbindungen der allgemeinen Formel I, bei denen $R^4$ Wasserstoff bedeutet, und die daraus gebildeten Acylierungsprodukte können im jeweiligen Lösungsmittel suspendiert oder gelöst vorliegen. Häufig liegt der größere Anteil dieser Verbindungen suspendiert vor. Nach Beendigung der Reaktion werden die gebildeten 4-Acylamino-pyrimidin-Derivate der allgemeinen Formel I ($R^4$ = Acyl) mittels üblicher Methoden kristallin isoliert und im Bedarfsfall durch Umkristallisation oder Chromatographie gereinigt.

Selbstverständlich können bei der Herstellung von Verbindungen der allgemeinen Formel I auch Substituenten im Phenylrest der Anilidgruppierung der Verbindungen der allgemeinen Formel I nach bekannten Methoden abgewandelt, d.h. in andere Substituenten überführt werden. So können beispielsweise Nitro- in Aminogruppen und diese wiederum in Acylaminogruppen oder Carboxyl- in Carbonestergruppen sowie umgekehrt Carbonester- in Carboxylgruppen überführt werden.

Erfindungsgemäß können außer den in den Ausführungsbeispielen beschriebenen Verbindungen beispielsweise auch die in der folgenden Tabelle 1 aufgeführten Verbindungen der allgemeinen Formel I bzw. deren Säureadditionssalze erhalten werden :

(Siehe die Formel, Seite 10)

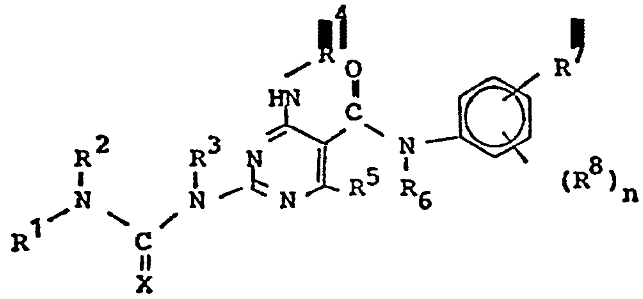

(I)

Tabelle 1

| | X | $R^1$ | $R^2$ | $R^3$ | $R^4$ | $R^5$ | $R^6$ | $R^7$ | $R^8$ | n |
|---|---|---|---|---|---|---|---|---|---|---|
| 1 | O | H | H | H | H | H | H | 3—$CF_3$ | — | 0 |
| 2 | O | H | H | H | H | H | $CH_2C_6H_5$ | H | — | 0 |
| 3 | O | H | H | H | $H_3CCO$ | H | $CH_2C_6H_5$ | H | — | 0 |
| 4 | O | H | H | H | H | H | $CH_2C_6H_5$ | 4—Cl | — | 0 |
| 5 | O | H | H | H | H | H | $CH_2C_6H_5$ | 4—Cl | 3—Cl | 1 |
| 6 | O | H | H | H | H | H | $CH_2C_6H_5$ | 3—$CF_3$ | — | 0 |
| 7 | O | H | H | H | H | H | 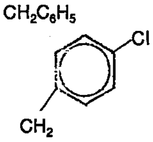 | H | — | 0 |
| 8 | O | H | $C_4H_9$ | H | H | H | $CH_3$ | 4—Cl | 3—$CF_3$ | 1 |
| 9 | O | H | H | H | H | H | $C_2H_5$ | 3—Cl | 2—$CH_3$ | 1 |
| 10 | O | H | H | H | H | H | $C_3H_5$ | 3—$OCH_3$ | 4—$OCH_3$ | 1 |
| 11 | O | H | $C_5H_{11}$ | H | H | H | $(H_3C)_2CH$ | 3—$CF_3$ | — | 0 |
| 12 | O | H | 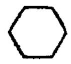 | H | H | H | $(H_3C)_2CH$ | 4—$HC(CH_3)_2$ | 3—Cl | 1 |
| 13 | O | H | $C_3H_7$ | H | H | H | $C_6H_5$ | 4—$HC(CH_3)_2$ | 3—Cl | 1 |
| 14 | O | H | $C_6H_{13}$ | H | H | H | $C_6H_5$ | 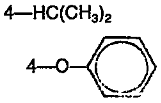 | 3—$CF_3$ | 1 |
| 15 | O | H | $CH_3$ | H | H | H | $C_3H_5$ | 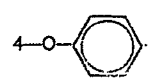 | 3—Cl<br>6—$CH_3$ | 2 |
| 16 | O | H | $C_6H_5$ | H | H | H | 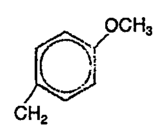 | 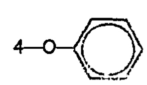 | — | 0 |
| 17 | O | H | $CH_3$ | H | H | H | $CH_2C_6H_5$ | 3—$CF_3$ | — | 0 |
| 18 | O | H | $CH_3$ | H | H | H | $CH(CH_3)_2$ | 4—$OC_2H_5$ | 3—$CF_3$ | 1 |
| 19 | O | $CH_3$ | $CH_3$ | H | H | H | $C_2H_5$ | 4—Cl | 3—$CF_3$ | 1 |
| 20 | O | $CH_3$ | $CH_3$ | H | H | H | 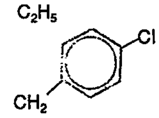 | 4—$HC(CH_3)_2$ | 3—Cl | 1 |
| 21 | O | $CH_3$ | $CH_3$ | H | H | H | $HC(CH_3)_2$ | 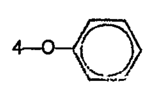 | 3—$CF_3$ | 1 |
| 22 | O | H | $C_2H_5$ | H | H | H | $CH_2C_6H_5$ | 4—Cl | 3—$CF_3$ | 1 |
| 23 | O | H | $C_7H_{15}$ | H | H | H | $C_2H_5$ | 3—$OCH_3$ | 4—$OCH_3$ | 1 |
| 24 | O | H | 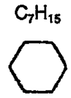 | H | H | H | $C_6H_5$ | 3—$CF_3$ | — | 0 |
| 25 | O | H | 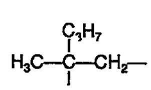 | H | H | H | H | 2—$OCH_3$ | 5—$CF_3$ | 1 |
| 26 | O | H | "  " | H | H | H | H | 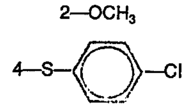 | 3—$CF_3$ | 1 |
| 27 | O | H | "  " | H | H | H | H | 4—$SCH_3$ | 3—$CF_3$ | 1 |

Tabelle 1 (Fortsetzung)

| | X | $R^1$ | $R^2$ | $R^3$ | $R^4$ | $R^5$ | $R^6$ | $R^7$ | $R^8$ | n |
|---|---|---|---|---|---|---|---|---|---|---|
| 28 | O | H | $(H_3C)_2C—CH_2—$ | | H | H | H | 2—$OC_6H_5$ | 5—$CF_3$ | 1 |
| 29 | O | H | " | " | H | H | H | 4—$SCH_3$ | 3—$CF_3$ | 1 |
| 30 | O | H | " | " | H | H | H | 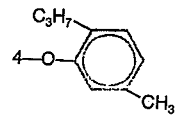 | 3—$CF_3$ | 1 |
| 31 | O | H | " | " | H | H | H | 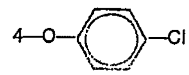 | 3—$CF_3$ | 1 |
| 32 | O | H | " | " | H | H | H | 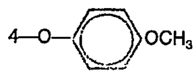 | 3—$CF_3$ | 1 |
| 33 | O | H | " | " | H | H | H | 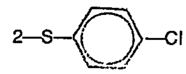 | 5—$CF_3$ | 1 |
| 34 | O | H | " | " | H | H | H | 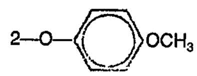 | 5—$CF_3$ | 1 |
| 35 | O | H | " | " | H | H | H | 4—$OC_4H_9$ | 3—$CF_3$ | 1 |
| 36 | O | H | $(H_3C)_2C—CH_2—$ | | H | H | H | 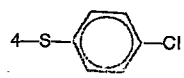 | 3—$CF_3$ | 1 |
| 37 | O | H | " | " | H | H | H | 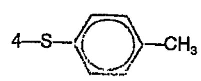 | 3—$CF_3$ | 1 |
| 38 | O | H | " | " | H | H | H | 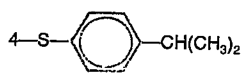 | 3—$CF_3$ | 1 |
| 39 | O | H | " | " | H | H | H | 2—$SC_4H_9$ | 5—$CF_3$ | 1 |
| 40 | O | H | " | " | H | H | H | 4—$SC_2H_5$ | 3—$CF_3$ | 1 |
| 41 | O | H | " | " | H | H | $CH_2C_6H_5$ | 4—$OC_6H_5$ | 3—$CF_3$ | 1 |
| 42 | O | H | " | " | H | H | H | 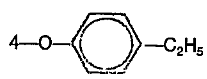 | 3—$CF_3$ | 1 |
| 43 | O | H | $(H_5C_2)_2C—CH_2—$ | | H | H | H | 5—$CF_3$ | 2—Cl | 1 |
| 44 | O | H | 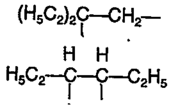 | | H | H | H | 3—$CF_3$ | — | 0 |
| 45 | O | H | 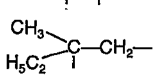 | | H | H | H | 5—$CF_3$ | 2—F | 1 |
| 46 | O | H | 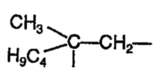 | | H | H | H | 3—$CF_3$ | — | 0 |
| 47 | O | H | 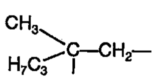 | | H | H | 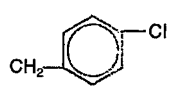 | 3—$CF_3$ | — | 0 |
| 48 | O | H | $(H_3C)_2C—CH_2—$ | | H | H | $CH_3$ | 3—$CF_3$ | 4—Cl | 1 |
| 49 | O | H | $(H_3C)_2C—CH_2—$ | | 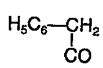 | H | H | 3—$CF_3$ | — | 0 |
| 50 | O | H | $C_4H_9$ | H | $H_7C_3CO$ | H | H | 4—$OC_2H_5$ | 3—Cl | 1 |
| 51 | S | $CH_3$ | $CH_3$ | H | H | H | H | 3—$CF_3$ | — | 0 |
| 52 | S | $CH_3$ | $CH_3$ | H | H | H | H | 4—$OC_6H_5$ | 2—Cl | 1 |
| 53 | S | H | $C_6H_5$ | H | H | H | H | 3—Cl | 2—$CH_3$ | 1 |
| 54 | S | H | $C_3H_5$ | H | H | H | $CH_2C_6H_5$ | 4—Cl | — | 0 |
| 55 | S | H | 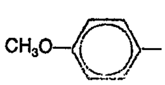 | H | H | H | H | 4—$OC_6H_5$ | — | 0 |

Tabelle 1 (Fortsetzung)

| | X | R¹ | R² | R³ | R⁴ | R⁵ | R⁶ | R⁷ | R⁸ | n |
|---|---|---|---|---|---|---|---|---|---|---|
| 56 | S | H | $C_3H_5$— | H | H | H | $CH_3$ | 4—$SCH_3$ | 3—Cl | 1 |
| 57 | O | H | $(H_3C)_2C$—$CH_2$— | | H | H | $CH_2CH{=}CH_2$ | 3—$CF_3$ | 6—F | 1 |
| 58 | O | H | $(H_3C)_2C$—$CH_2$— | | H | H | H | 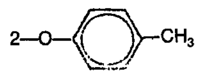 | 5—$CF_3$ | 1 |
| 59 | O | H | —$H_2C$—$CH_2$— | | H | H | $CH_2C_6H_5$ | 4—$NH_2$ | — | 0 |
| 60 | O | H | —$H_2C$—$CH_2$— | | H | H | H | 4—$OC_6H_5$ | 3—$CF_3$ | 1 |
| 61 | O | H | $C_3H_7$<br>$H_3C$—C—$CH_2$— | | H | H | H | 4—$CF_3$ | 2—F | 1 |
| 62 | O | H | " | " | H | H | H | 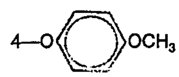 | 3—$CF_3$ | 1 |
| 63 | O | H | " | " | H | H | H | 2—$OC_6H_5$ | 5—$CF_3$ | 1 |
| 64 | O | —$(CH_2)_5$— | | H | H | H | H | 4—$OC_6H_5$ | — | 0 |
| 65 | O | —$(CH_2)_5$— | | H | H | H | H | 3—$CF_3$ | — | 0 |
| 66 | O | —$(CH_2)_5$— | | H | H | H | H | | 3,4—O—$CH_2$—O— | 1 |
| 67 | O | —$CH_2CH_2OCH_2CH_2$— | | H | H | H | H | | 3,4—O—$CH_2$—O— | 1 |
| 68 | O | —$CH_2CH_2OCH_2CH_2$— | | H | H | H | $CH_2C_6H_5$ | H | — | 0 |
| 69 | O | —$(CH_2)_4$— | | H | $CH_3CO$ | H | H | 4—$CH_3$ | 3—Cl | 1 |
| 70 | S | —$(CH_2)_4$— | | H | H | H | H | 3—$CF_3$ | — | 0 |
| 71 | S | —$(CH_2)_5$— | | H | H | H | $CH_2C_6H_5$ | H | — | 0 |
| 72 | O | H | $(H_3C)_2C$—$CH_2$— | | H | H | $CH_2C_6H_5$ | 3—$CF_3$ | — | 0 |
| 73 | O | H | H<br>$H_5C_6$—C—$CH_2$— | | H | H | H | 4—$COOC_2H_5$ | — | 0 |
| 74 | O | H | H<br>$H_5C_6$—C—$CH_2$— | | H | H | $CH_3$ | 3—$CF_3$ | — | 0 |
| 75 | O | H | 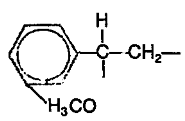 | | H | H | H | 3—$CF_3$ | — | 0 |
| 76 | O | H | $CH_3$<br>$H_5C_6$—C—$CH_2$— | | H | H | H | 3—$CF_3$ | — | 0 |
| 77 | O | H | $CH_3$<br>$H_5C_6$—C—$CH_2$— | | H | H | H | 3—$CF_3$ | 5—$CH_3$ | 1 |
| 78 | O | H | $(H_3C)_2C$——$CH_2$— | | H | H | H | 5—$CF_3$ | 2—Br | 1 |
| 79 | O | H | " | " | H | H | H | 3—$CF_3$ | 4—F | 1 |
| 80 | O | H | " | " | H | H | H | 5—$CF_3$ | 2—$SCH_3$ | 1 |
| 81 | O | H | " | " | H | H | H | 3—$CF_3$ | 4—$CH_3$ | 1 |
| 82 | O | H | " | " | H | H | H | 3—$CF_3$ | 2—Cl | 1 |
| 83 | O | H | " | " | H | H | H | 3—$CF_3$ | 2—F | 1 |
| 84 | O | H | " | " | H | H | H | 3—$CF_3$ | 5—F | 1 |
| 85 | O | H | " | " | H | H | H | 3—$CF_3$ | 5—Cl | 1 |
| 86 | O | H | " | " | H | H | H | 3—$CF_3$ | 5—$CH_3$ | 1 |
| 87 | O | H | " | " | H | H | H | 5—$CF_3$ | 2—$CH_3$ | 1 |
| 88 | O | H | " | " | H | H | H | 3—$CF_3$ | 4—Br | 1 |
| 89 | O | H | " | " | H | H | H | 3—$CF_3$ | 4—$OCH_3$ | 1 |
| 90 | O | H | " | " | H | H | H | 3—$CF_3$ | 5—$OCH_3$ | 1 |
| 91 | O | H | " | " | H | H | H | 5—$CF_3$ | 2—$OCH_3$ | 1 |
| 92 | O | H | " | " | H | H | H | 2—$OC(CH_3)_3$ | 5—$CF_3$ | 1 |
| 93 | O | H | " | " | H | H | H | 3—$CF_3$ | 4—$OC_2H_5$ | 1 |
| 94 | O | H | " | " | H | H | H | 5—$CF_3$ | 2—$OC_2H_5$ | 1 |
| 95 | O | H | " | " | H | H | H | 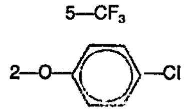 | 5—$CF_3$ | 1 |

Tabelle 1 (Fortsetzung)

| | X | $R^1$ | $R^2$ | $R^3$ | $R^4$ | $R^5$ | $R^6$ | $R^7$ | $R^8$ | n |
|---|---|---|---|---|---|---|---|---|---|---|
| 96 | O | H | $(H_3C)_2C—CH_2—$ | | H | H | H | 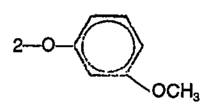 | 5—$CF_3$ | 1 |
| 97 | O | H | " | " | H | H | H | 4—$OC(CH_3)_3$ | 3—$CF_3$ | 1 |
| 98 | O | H | " | " | H | H | H | 2—S—$C_6H_5$ | 5—$CF_3$ | 1 |
| 99 | O | H | " | " | H | H | H | 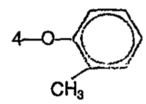 | 3—$CF_3$ | 1 |
| 100 | O | H | " | " | H | H | H | 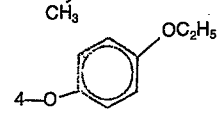 | 3—$CF_3$ | 1 |
| 101 | O | H | " | " | H | H | H | 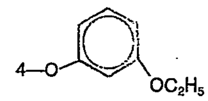 | 3—$CF_3$ | 1 |
| 102 | O | H | " | " | H | H | H | 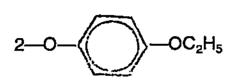 | 5—$CF_3$ | 1 |

Die erfindungsgemäßen neunen Verbindungen der allgemeinen Formel I besitzen wertvolle pharmakologische Eigenschaften und können daher als Arzneimittel verwendet werden. Sie zeichnen sich beispielsweise durch günstige Wirkungen auf den Fettstoffwechsel aus, insbesondere sind sie als Anorektika geeignet. Gegenüber den eingangs erwähnten ß-Phenylethylaminanalogen bzw. -derivaten und Verbindungen vom Mazindol-Typ zeichnen sie sich durch das Fehlen unerwünschter Nebenwirkungen auf das Herz-Kreislaufsystem und das Fehlen stimulierender Effekte auf das Zentralnervensystem aus und sind daher diesen Verbindungen überlegen. Da Übergewicht sehr häufig mit Störungen des Purinstoffwechsels (Serumharnsäureerhöhungen) oder Hyperlipidämien verbunden ist, sind zusätzliche antagonisierende Eigenschaften gegenüber diesen Stoffwechselentgleisungen erwünscht und von besonderem Vorteil. Einzelne Vertreter der allgemeinen Formel I zeigen auch diese güngstigen hypourikämischen Eigenschaften.

Die appetithemmende Wirkung der neuen Verbindungen der allgemeinen Formel I zeigt sich im pharmakologischen Test bei peroraler und/oder intraperitonealer Applikation an hungernden Ratten in einer Hemmung der Futteraufnahme und führt bei semichronischer Verabfolgung zu einer Hemmung der Körpergewichtszunahme.

Die Prüfung auf anorektische Wirkung (Futterverbrauchshemmung) der Verbindungen im akuten Versuch an der Ratte erfolgte durch Messung des Futterverbrauches von 48 h hungernden Ratten über einen Zeitraum von 6 Stunden im stündlichen Intervall. Zur Verwendung kamen männliche Raten des Stammes Wistar mit einem Körpergewicht über 110 g, die mehrere Tage in den Versuchsräumen aklimatisiert wurden. Sie hatten freien Zugang zu Futter und Wasser bis zum Versuchsbeginn. 48 Stunden vor der Applikation der Substanz wurde ihnen das Futter entzogen. Dann wurde ihnen die zu prüfende Substanz entweder oral (mit der Schlundsonde) oder intraperitoneal in 1 %iger Tylose mit einem Volumen von 0,5 ml/100 g Körpergewicht appliziert. Eine halbe Stunde nach der intraperitonealen bzw. 1 Stunde nach der oralen Applikation wurde den Tieren eine genau abgemessene Menge pelletierten Futters angeboten und die Menge des gefressenen Futters stündlich über 6 Stunden durch Rückwiegen der vorgelegten Futtermenge bestimmt. Pro Verbindung und Dosierung wurden in der Regel 6 Ratten verwendet, die einzeln in Makrolonkäfigen des Typus 3 während der Versuchszeit gehalten wurden.

Aus dem Futterverbrauch jedes einzelnen Tieres wurde zu jedem Zeitpunkt der Futterverbrauchsbestimmung der Mittelwert der Gruppe gebildet. Dieser Mittelwert des Futterverbrauches wurde mit demjenigen einer mitlaufenden Kontrollgruppe, die nur das Lösungs- bzw. Suspensionsmittel erhalten hatte, verglichen. Die Veränderungen gegenüber der Kontrollgruppe sind in den nachfolgenden Tabellen I und II in % angegeben.

(Siehe die Tabellen, Seite 14)

Tabelle I

| Verbindung gemäß Beispiel | Dosis mg/kg | % Futterverbrauch gegenüber Kontrollgruppe nach Stunden | | | | | |
|---|---|---|---|---|---|---|---|
| | | 1 | 2 | 3 | 4 | 5 | 6 |
| 3 | 0,3 | − 35 | − 25 | − 13 | − 10 | − 6 | − 15 |
| | 0,1 | − 27 | − 13 | − 9 | − 11 | − 12 | − 14 |
| 23 | 1 | − 26 | − 25 | − 43 | − 28 | − 24 | − 22 |
| 15 | 3 | − 70 | − 49 | − 38 | − 28 | − 21 | − 26 |
| | 1 | + 5 | − 4 | − 26 | − 28 | − 19 | − 16 |
| 1 | 10 | − 37 | − 37 | − 30 | − 40 | − 43 | − 35 |

Hemmung der Futteraufnahme (in % gegenüber einer Kontrollgruppe) 48 Stunden hungernder Ratten über den Zeitraum von 1 bis 6 Stunden. Interperitoneale Applikation 1/2 Stunde vor Futterangebot.

Tabelle II

| Verbindung gemäß Beispiel | Dosis mg/kg | % Futterverbrauch gegenüber Kontrollgruppe nach Stunden | | | | | |
|---|---|---|---|---|---|---|---|
| | | 1 | 2 | 3 | 4 | 5 | 6 |
| 3 | 10 | − 27 | − 13 | − 15 | − 17 | − 10 | − 11 |
| | 3 | − 26 | − 8 | − 15 | − 16 | − 9 | − 10 |
| 23 | 3 | − 49 | − 17 | − 24 | − 28 | − 12 | − 10 |
| 15 | 3 | − 12 | − 6 | − 27 | − 21 | − 14 | − 14 |
| 1 | 10 | − 74 | − 49 | − 47 | − 47 | − 39 | − 36 |

Hemmung der Futteraufnahme (in % gegenüber einer Kontrollgruppe) 48 Stunden hungernder Ratten über den Zeitraum von 1 bis 6 Stunden. Perorale Applikation 1 Stunde vor Futterangebot.

Die günstige Wirkung auf den Fettstoffwechsel im Sinne einer hypolipidämischen und/oder hypourikämischen Wirkung zeigt sich an Ratten, die mehrere Tage mit den erwähnten Verbindungen oral behandelt werden und denen vor und nach der Behandlung Blut entnommen wurde. In dem daraus gewonnenen Serum werden Cholesterin, nach der CHOD-PAP-Methode (Boehringer), Triglyceride nach Eggstein und Kreutz und Harnsäure nach Kortümn M. und Kling, O. [Ärztl. Lab. *18*, 33, (1972)] enzymatisch bestimmt und die Veränderungen gegenüber dem Anfangswert oder gegenüber einer mitlaufenden lediglich mit dem Lösungs- bzw. Suspensionsmittel behandelten Kontrollgruppe verglichen.

Bei weiteren Untersuchungen betreffend die Beeinflussung des Lipidstoffwechsels durch erfindungsgemäße Verbindungen wurde beobachtet, daß sie die Konzentration der atherogenen Lipoproteinfraktionen LDL und VLDL im Serum stark vermindern, während sie den Schutzfaktor HDL nicht beeinflussen oder nur in ganz geringem Ausmaß erniedrigen. Demzufolge wird das Verhältnis HDL zu den atherogenen Fraktionen um ein Mehrfaches erhöht (bezogen auf eine ungehandelte Kontrollgruppe). Im Vergleich dazu erniedrigt der bekannte Lipidsenker Clofibrat HDL-Cholesterin in einem wesentlich stärkeren Ausmaß als LDL- und VLDL-Cholesterin. Auch das Clofibrat-Analoge Bezafibrat erniedrigt HDL-Cholesterin fast im gleichem Ausmaß wie LDL-Cholesterin. Außerdem erhöhen Clofibrat und seine Analogen stark das relative Lebergewicht, während die neuen Verbindungen diesbezüglich neutral sind.

Die Versuche wurden, wie nachfolgend beschrieben, durchgeführt : Männliche Ratten des Stammes HOE : WISKf (SPF 71) mit einem Ausgangsgewicht über 220 g, in Gruppen von je 10 Tieren, erhielten täglich einmal (morgens) die angegebene Dosierung der zu prüfenden Verbindung in PEG 400 per Schlundsonde ; die Kontrollgruppe erhielt nur PEG 400. Insgesamt wurden 7 bzw. 28 Applikationen

verabreicht, die letzte (7. bzw. 28. Applikation) 24 Stunden vor Blutentnahme und Tötung. Zu Futter und Wasser bestand freier Zugang während des Versuches. 24 Stunden vor der Blutentnahme, die retroorbital unter leichter Äthernarkose erfolgte, wurde das Futter entzogen. Unmittelbar nach der Blutentnahme wurden die Tiere durch Distorsion der Wirbelsäule getötet. Es wurden die Lebern entnommen und das relative Lebergewicht bestimmt. Außerdem wurde die Körpergewichtsentwicklung und der Futterverbrauch untersucht.

Zur Analyse der Serum-Lipoproteine wurde das Serum aller Ratten einer Gruppe gepoolt. Die Serum-Lipoproteine wurden mit der präparativen Ultrazentrifuge (Beckman L 250 B, Rotor FW 50 Ti) in VLDL, LDL und HDL getrennt.

Zur enzymatischen Bestimmung des Cholesterins nach der CHOP-PAP-Methode (RÖSCHLAU, P., BERNDT, E. und W. GRUBER : 9th int. Congr. on klin. Chemistry, Toronto, 1975, Abstract Nr. 1) sowie der Triglyceride [EGGSTEIN, M. und KREUTZ, F.H. : Klin. Wschr. *44,* 262 und 267 (1966) ; WAHLEFELD, A.W., in H.O. BERGMEIER : Methoden der enzymatischen Analyse 3. Auflage, Band II, Verlag Chemie 1974, S. 1978] in den getrennten Lipoproteinfraktionen wurde eine Testkombination von BOEHRINGER/Mannheim verwendet, die Bestimmung des Proteins erfolgte nach der Methode von LOWRY et al. [LOWRY, O.H., ROSEBOROUGH, N.J., FARR, A.L., and R.J. RANDELL : J. Biol. Chem. *193,* 265 (1951)].

Tabelle III

| Verbindung | Dosis mg/kg/ Tag | Tier-zahl | % Veränderung gegenüber Kontrollgruppe von: CHOLESTERIN in VLDL | CHOLESTERIN in LDL | CHOLESTERIN in HDL | PROTEIN in VLDL | PROTEIN in LDL | PROTEIN in HDL | HDL / VLDL + LDL Cholesterin | HDL / VLDL + LDL Protein | relatives Leber-gewicht | Körper-gewicht | Futter-verbrauch |
|---|---|---|---|---|---|---|---|---|---|---|---|---|---|
| gemäß | 30 | 10 | − 100 | − 80 | − 14 | − 40 | − 39 | + 1 | 580 | 150 | − 5 | − 6 | − 10 |
| Beispiel 1 | 20 | 10 | − 87 | − 56 | − 25 | − 43 | − 30 | + 7 | 200 | 170 | − 5 | − 9 | − 21 |
| | 5 | 10 | 0 | − 25 | + 2 | 0 | − 7 | + 5 | 120 | 100 | − 2 | 0 | − 9 |
| gemäß Beispiel 67 | 30 | 10 | − 100 | − 82 | − 25 | − 38 | − 48 | + 3 | 1 130 | 180 | 0 | − 12 | − 56 |
| Clofibrat | 100 | 10 | + 12 | − 16 | − 36 | + 8 | − 3 | + 8 | 130 | 130 | + 28 | | |
| Bezafibrat | 50 | 10 | 0 | − 31 | − 21 | + 23 | − 23 | + 29 | 110 | 150 | + 39 | | |

Beeinflussung der Serumlipoproteine, des relativen Lebergewichtes, der Körpergewichtsentwicklung und des Futterverbrauches von ♂ Ratten nach *7-tätiger* oraler Behandlung.

Tabelle IV

| Verbindung | Dosis mg/kg/ Tag | Tier-zahl | % Veränderung gegenüber Kontrollgruppe von: CHOLESTERIN in VLDL | CHOLESTERIN in LDL | CHOLESTERIN in HDL | PROTEIN in VLDL | PROTEIN in LDL | PROTEIN in HDL | GLYCERIN in VLDL | GLYCERIN in HDL | GLYCERIN in LDL | relatives Leberge-wicht | HDL / VLDL + LDL Cholesterin | HDL / VLDL + LDL Protein |
|---|---|---|---|---|---|---|---|---|---|---|---|---|---|---|
| gemäß | 30 | 10 | − 100 | − 36 | − 3 | − 38 | − 17 | − 5 | − 60 | − 2 | + 9 | + 9 | 180 | 130 |
| Beispiel 1 | 3 | 10 | − 58 | − 2 | − 1 | − 19 | − 4 | − 3 | − 33 | − 8 | − 7 | 0 | 130 | 110 |

Beeinflussung der Serumlipoproteine und des relativen Lebergewichtes nach *4-wöchiger* oraler Behandlung von ♂ Wistar-Ratten.

Die Verbindungen können daher als Appetitzügler zur Behandlung von Fettsucht allein oder in Begleitung anderer Fettstoffwechselstörungen eingesetzt werden. Die täglich zu verabreichende Dosis beträgt 2 bis 2 000 mg, vorzugsweise 2 bis 200 mg, wobei diese Menge zweckmäßigerweise in kleineren Dosen von 0,2 bis 50 mg täglich zwei bis viermal oder in Retardform verabreicht wird.

Die neuen Verbindungen der allgemeinen Formel I können in Form der freien Basen und/oder in Form ihrer pharmazeutisch annehmbaren Säureadditionssalze aufgrund ihrer pharmakologischen Eigenschaften als Arzneimittel Verwendung finden, wobei man sie entweder allein oder vermischt mit geeigneten Trägerstoffen und/oder verträglichen Verdünnungsmitteln und gegebenenfalls auch noch mit anderen Zusätzen verabreicht.

Gegenstand der Erfindung sind somit auch Arzneimittel, insbesondere zur Behandlung der Adipositas, die aus wenigstens einer Verbindung der allgemeinen Formel I, gegebenenfalls in Form von deren physiologisch verträglichen Salzen bestehen oder diesen Wirkstoff enthalten. Sie sind gewöhnlich neben den üblichen pharmazeutisch akzeptablen Trägerstoffen und/oder Verdünnungsmitteln vorhanden. Die Präparate können oral, rectal oder parenteral verabreicht werden. Geeignete feste oder flüssige galenische Zubereitungsformen sind beispielsweise Tabletten, Dragees, Pulver, Kapseln, Suppositorien, Sirupe, Emulsionen, Suspensionen, Tropfen oder injizierbare Lösungen sowie Präparate mit protrahierter Wirkstoff-Freigabe. Als häufig verwendete Trägerstoffe bzw. Verdünnungsmittel seien z.B. verschiedene Zucker oder Stärkearten, Cellulosederivate, Magnesiumcarbonat, Gelatine, tierische und pflanzliche Öle, Polyethylenglykole, Wasser oder andere geeignete Lösungsmittel genannt.

Vorzugsweise können die Präparate in Dosierungseinheiten hergestellt werden. Insbesondere Tabletten, Kapseln, Suppositorien und Ampullen stellen Beispiele für geeignete Dosierungseinheiten dar. Jede Dosierungseinheit kann bis zu 1 000 mg, bevorzugt jedoch 1 bis 200 mg des aktiven Bestandteils enthalten.

Die in den nachfolgenden Beispielen angegebenen, nicht korrigierten Schmelzpunkte (Fp.) sind in den meisten Fällen Zersetzungspunkte. Die IR-Spektren wurden mit einem Perkin Elmer-Spektrophotometer, Modell 157 (NaCl-Prisma), in KBr aufgenommen, die angegebenen IR-spektroskopischen Daten sind jeweils auf eine Polystyrol-Eichbande bei 6,24 μ bezogen.

## Beispiel 1

Eine Mischung aus 14,2 g (50 mMol) Ethoximethylen-cyanacet(3-trifluormethyl-anilid), 50 ml 1,2-Dimethoxiethan, 8,6 g (55 mMol) 1-Amidino-4,4-dimethyl-imidazolidin-2-on und 50 ml Isopropanol wurde 30 min bei Raumtemperatur und 1,5 Stdn. unter Rückflußkochen gerührt. Nach dem Abkühlen saugte man den Feststoff ab und wusch diesen mit Aceton aus. Anschließend wurde der kristalline Stoff aus 800 ml Ethanol umkristallisiert und dabei die heiße Lösung filtriert. Man erhielt 10,5 g (53,3 %) reines 4-Amino-2-(4,4-dimethyl-imidazolidin-2-on-1-yl)-pyrimidin-5-carbonsäure-(3-trifluormethyl-anilid) vom Fp. 293-4 °C, IR : (Ureido)-C = O 5,82 μ. Aus den vereinigten Mutterlaugen wurden durch Kristallisation aus Ethanol weitere 7,3 g (37 %) reines Produkt vom Fp. 293-4 °C erhalten.

Analyse : $C_{17}H_{17}F_3N_6O_2$

| | | | | | |
|---|---|---|---|---|---|
| ber. (%) : | C 51,8 ; | H 4,3 ; | F 14,4 ; | N 21,3 | MG 394,4 |
| gef. (%) : | C 51,7 ; | H 4,3 ; | | N 21,0 | |

## Beispiel 2

Eine Mischung aus 14,2 g (50 mMol) Ethoximethylen-cyanacet-(3-trifluormethyl-anilid), 8,6 g (55 mMol) 1-Amidino-4,4-dimethyl-imidazolidin-2-on und 80 ml Acetonitril wurde 20 min, bei 40 °C und 2 Stdn. unter Rückflußkochen gerührt, auf 15 °C gekühlt und abgesaugt. Der isolierte kristalline Stoff (10,8 g) stellte laut DC quasi reines 4-Amino-2-(4,4-dimethyl-imidazolidin-2-on-1-yl)-pyrimidin-5-carb-(3-trifluormethyl-anilid) (Schmp. 292-93 °C) dar. Nach Einengen des Filtrats und Umkristallisieren des dabei erhaltenen Feststoffs aus Acetonitril erhielt man weitere 7,2 g (Fp. 293-4 °C) des oben genannten Pyrimidinderivats, womit die Ausbeute 91,5 % betrug.

Ein analog ausgeführter Ansatz, bei dem anstelle von Acetonitril abs. Ethanol als Lösungsmittel benutzt wurde, lieferte nach Isolierung des primär anfallenden Feststoffs und Aufarbeitung der ethanolischen Mutterlaugen durch Einengen und Umkristallisieren der weiteren, erhaltenen kristallinen Substanzen insgesamt 88,5 % d.Th. an 4-Amino-2-(4,4-dimethyl-imidazolidin-2-on-1-yl)-pyrimidin-5-carb-(3-trifluormethylanilid) vom Fp. 293-4 °C.

## Beispiel 3

Eine Mischung aus 13,25 g (50 mMol) Ethoximethylen-cyanacet-(3-chlor-2-methyl-anilid), 8,7 g (56 mMol) 1-Amidino-4,4-dimethyl-imidazolidin-2-on, 100 ml 1,2-Dimethoxiethan und 50 ml Isopropanol wurde je 30 min bei Raumtemperatur und bei 60 °C und 1,5 Stdn. unter Rückflußkochen gerührt, anschließend auf 15 °C abgekühlt und abgesaugt. Den Filterrückstand wusch man mit Aceton nach und trocknete ihn bei 90 °C im Vakuum. Man erhielt 18,25 g (97,3 %) 4-Amino-2-(4,4-dimethyl-imidazolidin-2-on-1-yl)-pyrimidin-5-carbonsäure-(3-chlor-2-methyl-anilid) vom Fp. 287-8 °C,
IR : (Ureido)-C = O : 5,80 μ.

Analyse : $C_{17}H_{19}ClN_6O_2$

| | | | | | |
|---|---|---|---|---|---|
| ber. (%) : | C 54,5 ; | H 5,1 ; | Cl 9,5 ; | N 22,4 | MG 374,9 |
| gef. (%) : | C 54,2 ; | H 5,1 ; | Cl 9,8 ; | N 22,2 | |

Ein in analoger Weise ausgeführter Ansatz, bei dem anstelle von Dimethoxiethan und Isopropanol nur 100 ml Isopropanol als Lösungs- bzw. Verdünnungsmittel benutzt wurden, lieferte nach Aufarbeitung

der Mutterlaugen insgesamt 17,8 g (94,5 %) reines 4-Amino-2-(4,4-dimethyl-imidazolidin-on-1-yl)-pyrimidin-5-carb-(3-chlor-2-methyl-anilid) vom Fp. 286-8 °C.

Beispiel 4

Eine Mischung aus 13,25 g (50 mMol) Ethoximethylen-cyanacet-(3-chlor-4-methyl-anilid), 8,6 (55 mMol) 1-Amidino-4,4-dimethyl-imidazolidin-2-on, 100 ml 1,2-Dimethoxiethan und 50 ml Isopropanol wurde 30 min bei Raumtemperatur und 2 Stdn. unter Rückflußkochen gerührt, anschließend auf 15 °C gekühlt und abgesaugt. Den Filterrückstand wusch man mit Aceton nach und trocknete ihn bei 90 °C im Vakuum. Dabei wurden 17,4 g (93 %) 4-Amino-2-(4,4-dimethyl-imidazolidin-2-on-1-yl)-pyrimidin-5-carbonsäure-(3-chlor-4-methyl-anilid) vom Fp. 290-2 °C erhalten. Nach Einengen des Filtrats wurden weitere 1,27 g (6,8 %) dieses Produkts mit einem Fp. von 292-3 °C (Zers.) erhalten,

IR : (Ureido)-C = O : 5,76 μ.

Analyse : $C_{17}H_{19}ClN_6O_2$

ber. (%) : C 54,5 ; H 5,1 ; Cl 9,5 ; N 22,4 MG 374,9

gef. (%) : C 54,0 ; H 5,1 ; Cl 9,4 ; N 22,2

Entsprechend dieser Arbeitsweise wurden die in der folgenden Tabelle 2 aufgeführten 4-Amino-2-(imidazolidin-2-on-1-yl)-pyrimidin-5-carbanilide hergestellt (Beispiele 5-23).

Tabelle 2 *

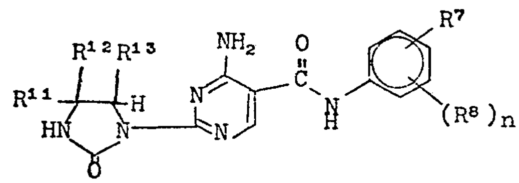

| Beispiel Nr. | $R^{11}$ | $R^{12}$ | $R^{13}$ | $R^7$ | $R^8$ | n | Fp. °C** | Ausbeute in % |
|---|---|---|---|---|---|---|---|---|
| 5 | H | H | H | 4—Cl | — | 0 | 236-7 | 83 |
| 6 | $CH_3$ | H | H | 3—$CF_3$ | — | 0 | 299-300 | 83 |
| 7 | $CH_3$ | H | H | 3—Cl | 2—$CH_3$ | 1 | 252-3 | 68** |
| 8 | $CH_3$ | H | H | 3—Cl | 4—$OC_2H_5$ | 1 | 231-2 | 87 |
| 9 | $CH=CH_2$ | H | H | 3—$CF_3$ | — | 0 | 271-3 | 80 |
| 10 | $CH_3$ | H | $CH_3$ | 3—$CF_3$ | — | 0 | 272-4 | 42 |
| 11 | $CH_3$ | $CH_3$ | H | 4—Cl | — | 0 | 291-2 | 85 |
| 12 | $CH_3$ | $CH_3$ | H | 3—Cl | 4—$OC_2H_5$ | 1 | 266-8 | 86 |
| 13 | $CH_3$ | $CH_3$ | H | 4—O— 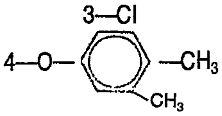 | 3—Cl | 1 | 260-1 | 95 |
| 14 | $CH_3$ | $CH_3$ | H | 4—$CH(CH_3)_2$ | 3—Cl | 1 | 303-5 | 94 |
| 15 | $CH_3$ | $CH_3$ | H | 4—Cl | 2—$CH_3$ | 1 | 277-8 | 84 |
| 16 | $CH_3$ | $CH_3$ | H | 4—Cl | 3—$CH_3$ | 1 | 282-4 | 95 |
| 17 | $CH_3$ | $CH_3$ | H | 2—Cl | 6—$CH_3$ | 1 | 165-73 | 80 |
| 18 | $CH_3$ | $CH_3$ | H | 5—Cl | 2—$CH_3$ | 1 | 242-4 | 84 |
| 19 | $CH_3$ | $CH_3$ | H | 2—Cl | 4—$NO_2$ | 1 | 286-8 | 72 |
| 20 | $CH_3$ | $CH_3$ | H | 4—$CH(CH_3)_2$ | H | 0 | 299-300 | 97 |
| 21 | $CH_3$ | $CH_3$ | H | 2—$CH_3$ | 4—$CH_3$ 6—$CH_3$ | 2 | 218-20 | 58 |
| 22 | $CH_3$ | $CH_3$ | H | 2—$OCH_3$ | 4—$OCH_3$ 5—$OCH_3$ | 2 | 240-1 | 80 |
| 23 | $CH_3$ | $CH_3$ | H | 4—$OC_6H_5$ | 3—Cl 6—$CH_3$ | 2 | 262-3 | 89 |
| 24 a | $CH_3$ | $CH_3$ | H | 3.4—O—$CH_2$—O— | | 1 | 310-11 | 70 |
| 24 b | $CH_3$ | $CH_3$ | H | 4—$COOC_2H_5$ | — | 0 | 294-5 | 69 |

* Die gemäß den Beispielen 5-24 b erhaltenen Verbindungen ergaben die den Bruttoformeln entsprechenden korrekten Analysenwerte. Im IR-Spektrum enthalten diese Verbindungen eine charakteristische, intensive Bande des Imidazolidinon-Carbonyls bei 5,76-5,82 μ.

** Bei den Schmelzpunkten handelt es sich in der Mehrzahl der Fälle um Zersetzungspunkte

** (4-R)-Konfiguration im Imidazolidin-Ring.

Beispiel 25

Zu einer Mischung aus 4,74 g (20 mMol) 1-Amidino-4,5-dimethyl-imidazolidin-2-on-hydrobromid (threo-erythro-Mischung), 5,30 g (20 mMol) Ethoximethylen-cyanacet-(3-chlor-4-methyl-anilid) und 80 ml abs. 1,2-Dimethoxiethan gab man anteilweise bei Raumtemperatur unter Rühren in 15 min 0,65 g (22 mMol) 80 %-ige Natriumhydrid-Paraffinoel-Suspension. Man rührte 20 min bei Raumtemperatur und 2 Stdn. unter Rückflußkochen nach. Anschließend saugte man den Feststoff ab und wusch diesen mit Wasser nach. Das Filtrat wurde im Vakuum eingedampft, den Rückstand vermischte man mit Aceton und saugte den Feststoff ab. Beide Feststoffanteile (6,60 g) wurden vereinigt und mit 300 ml Methanol ausgekocht und filtriert. Der Methanolextrakt wurde im Vakuum eingedampft und der verbleibende Rückstand wurde aus Aceton kristallisiert. Das Kristallisat (4,0 g) wurde mit einer Lösung von 2,6 g $NaHCO_3$ in 90 ml Wasser versetzt und 24 Stdn. geschüttelt. Anschließend saugte man ab, wusch mit Wasser aus und trocknete den Feststoff. Man erhielt so 3,30 g (44 %) reines 4-Amino-2-(4,5-dimethyl-imidazolidin-2-on-1-yl)-pyrimidin-5-carbonsäure-(3-chlor-4-methylanilid) vom Fp. 252-3 °C ;
IR : (Ureido)-CO : 5,80 μ.

Analyse : $C_{17}H_{19}ClN_6O_2$
ber. (%) : C 54,5 ; H 5,1 ; Cl 9,5 ; N 22,4 MG 374,9
gef. (%) : C 54,2 ; H 5,1 ; Cl 9,8 ; N 22,7 MG 374 , 376 (massenspektroskopisch)

Beispiel 26

Zu einer Lösung von 0,69 g Natrium in 50 ml abs. Ethanol gab man bei -2 bis 0 °C anteilweise 7,1 g (30 mMol) 1-Amidino-4,5-dimethyl-imidazolidin-2-on-hydrobromid (threo-erythro-Mischung), rührte 15 min bei 20-23 °C nach und fügte dann eine Suspension von 8,0 g (30 mMol) Ethoximethylen-cyanacet-(3-chlor-2-methyl-anilid) in 50 ml 1,2-Dimethoxiethan zu. Nach 15 min Rühren bei Raumtemperatur und 2 Stdn. unter Rückflußkochen wurde die Reaktionslösung im Vakuum eingedampft. Den Rückstand versetzte man mit einer Lösung von 6 g $NaHCO_3$ in 200 ml Wasser, schüttelte 8 Stdn. bei Raumtemperatur und saugte anschließend den Feststoff ab und wusch mit Wasser nach und trocknete das Produkt. Auf diese Weise wurden 8,2 g (73 %) 4-Amino-2-(4,5-dimethyl-imidazolidin-2-on-1-yl)-pyrimidin-5-carbonsäure-(3-chlor-2-methyl-anilid) vom Fp. 226-7 °C ;
(IR : (Ureido)-CO : 5,85 μ) erhalten.

Analyse : $C_{17}H_{19}ClN_6O_2$
gef. (%) : C 54,2 ; H 5,1 ; Cl 9,8 ; N 22,0 MG 374 ; 376 (massenspektroskopisch)

Beispiel 27

Zu einer Lösung von 4,0 g (ca. 15 mMol) rohem, öligen 1-Amidino-4-methyl-4-propyl-imidazolidin-2-on-hydrobromid in 10 ml abs. Ethanol tropft man bei -3 bis 0 °C eine Lösung von 0,28 g (12 mg-Atom) Natrium in 18 ml abs. Ethanol, rührte 15 min bei Raumtemperatur, gab dann eine Suspension von 3,52 g (12 mMol) Ethoximethylen-cyanacet-(3-chlor-4-isopropyl-anilid) in 17 ml 1,2-Dimethoxiethan zu und rührte 20 min bei Raumtemperatur und 3 Stdn. unter Rückflußkochen. Nach dem Abkühlen wurde abgesaugt und der Filterrückstand mit Aceton nachgewaschen. Das Filtrat wurde im Vakuum eingedampft und der verbleibende Rückstand in Wasser suspendiert, abgesaugt und mit Wasser ausgewaschen. Beide Feststoffanteile wurden vereinigt und mit einer Lösung von 2,5 g $NaHCO_3$ in 100 ml Wasser 3 Stdn. geschüttelt. Anschließend saugte man ab, wusch den Feststoff mit Wasser und kristallisierte ihn nach dem Trocknen aus Methanol um. Zusammen mit einem nach Einengen der Mutterlauge erhaltenen zweiten Kristallisat wurden auf diese Weise 4,80 g (93% bzw. auf 12 mMol) reines 4-Amino-2-(4-methyl-4-propyl-imidazolidin-2-on-1-yl)-pyrimidin-5-carbonsäure-(3-chlor-4-isopropyl-anilid) vom Fp. 252-4 °C erhalten.

Analyse : $C_{21}H_{27}ClN_6O_2$
ber. (%) : C 58,5 ; H 6,3 ; Cl 8,2 ; N 19,5 MG 430,9
gef. (%) : C 58,4 ; H 6,3 ; Cl 8,4 ; N 19,6

Beispiel 28

Nach der gleichen Arbeitsweise wie im Beispiel 27 beschrieben wurde ausgehend von 10,9 g (41 mMol) rohem, öligen 1-Amidino-4-methyl-4-propyl-imidazolidin-2-on-hydrobromid und 8,75 g (33 mMol) Ethoximethylen-cyanacet-(3-chlor-2-methyl-anilid) 10,4 g (78 % bezogen auf 33 mMol) reines 4-Amino-2-(4-methyl-4-propyl-imidazolidin-2-on-1-yl)-pyrimidin-5-carbonsäure-(3-chlor-2-methyl-anilid) vom Fp. 252-3 °C erhalten.

Analyse : $C_{19}H_{23}ClN_6O_2$
ber. (%) : C 56,6 ; H 5,8 ; Cl 8,8 ; N 20,9 MG 402,9
gef. (%) : C 56,3 ; H 5,8 ; Cl 8,6 ; N 20,4 MG 402 ; 404 (massenspektroskopisch)

Beispiel 29

Eine Mischung (Lösung) aus 9,6 g (62 mMol) 1-Amidino-4,4-dimethyl-imidazolidin-2-on, 18,4 g (60 mMol) rohem, öligen Ethoximethylen-cyanacet-N-benzyl-anild, 180 ml Dimethoxiethan und 120 ml Isopropanol wurde 20 min bei Raumtemperatur und 3 Stdn. unter Rückflußkochen gerührt und danach im Vakuum eingedampft. Der ölige Rückstand (27 g) wurde aus 80 ml $CH_2Cl_2$ auf eine 43 cm hohe (∅ = 5,0 cm)

Kieselgel S (0,063-0,2 mm, Riedel-DeHäen AG)-$CH_2Cl_2$-Säule aufgezogen und chromatographiert. Nach Elution mit 1 200 ml $CH_2Cl_2$, je 600 ml $CH_2Cl_2/C_2H_5OH$ 100 : 1, 100 : 2, 100 : 3 und 100 : 4 wurden mit je 600 ml $CH_2Cl_2/C_2H_5OH$ 100 : 5 und 100 : 6 4,40 g 5-Cyano-2-(4,4-dimethyl-imidazolidin-2-on-1-yl)-3,4-dihydro-4-oxo-pyrimidin (Fp. 273-4 °C) und bei weiterer Elution mit 200 ml $CH_2Cl_2/C_2H_5OH$ 100 : 7 und je 600 ml 100 : 8 und 100 : 9 3,50 g Produkt eluiert, in dem die gewünschte Verbindung stark angereichert war. Diese 3,5 g wurden aus Methanol umkristallisiert, wobei 2,60 g (10,5 %) reines 4-Amino-2-(4,4-dimethyl-imidazolidin-2-on-1-yl)-pyrimidin-5-carbonsäure-N-benzyl-anilid vom Fp. 276-7 °C ;

(IR : (Ureido)-CO 5,79 μ) erhalten wurden.

Analyse : $C_{23}H_{24}N_6O_2$
ber. (%) : C 66,3 ; H 5,8 ; N 20,2 MG 416,5
gef. (%) : C 65,9 ; H 5,7 ; N 19,8 MG 416,0 (massenspektroskopisch)

Beispiel 30

Eine Mischung aus 5,78 g (30 mMol) 1-Amidino-4,4-dimethylimidazolidin-2-on-hydrochlorid, 2,30 g (16,5 mMol) $K_2CO_3$, 70 ml Acetonitril und 7,5 g (30 mMol) Ethoximethylen-cyanacet-(4-chloranilid) wurde 1 Std. bei 60 °C und 5 Stdn. unter Rückflußkochen gerührt und anschließend im Vakuum eingedampft. Den Rückstand versetzte man mit 70 ml Wasser, schüttelte durch, saugte den Feststoff ab und wusch diesen mit Wasser und Aceton aus. Nach dem Trocknen wurden 8,33 g (77 %) 4-Amino-2-(4,4-dimethyl-imidazolidin-2-on-1-yl)-pyrimidin-5-carb-(4-chloranilid) vom Fp. 290-1 °C erhalten.

Beispiel 31

Eine Mischung aus 2,37 g (10 mMol) 1-Amidino-4,4-dimethylimidazolidin-2-on-hydrobromid, 1,10 g Triethylamin, 60 ml Methylenchlorid und 2,60 g (10 mMol) Ethoximethylencyanacet-(4-isopropyl-anilid) wurde 4 Stdn. unter Rückfluß gekocht. Anschließend wurde das Lösungsmittel verdampft. Den Rückstand versetzte man mit 50 ml Wasser, schüttelte 10 min, saugte den Feststoff ab, wusch ihn mit Wasser und versetzte ihn danach mit einer Lösung von 1,7 g $NaHCO_3$ in 50 ml Wasser und schüttelte 3 Stdn. bei Raumtemperatur. Danach wurde abgesaugt und der Filterrückstand wurde nacheinander mit Wasser und Aceton gewaschen und getrocknet. Man erhielt 2,91 g (79 %) reines 4-Amino-2-(4,4-dimethylimidazolidin-2-on-1-yl)-pyrimidin-5-carb-(4-isopropylanilid) vom Fp. 299-300 °C.

Beispiel 32

Zu einer Lösung von 4,0 g (ca. 15 mMol) rohem, öligen 1-Amidino-4,4-diethyl-imidazolidin-2-on-hydrobromid in 10 ml abs. Ethanol tropft man bei – 5 bis 0 °C eine Lösung von 0,28 g (12 mg-Atom) Natrium in 18 ml abs. Ethanol, rührte 15 min bei 23 °C, gab dann eine Suspension von 3,24 g (10 mMol) Ethoximethylen-cyanacet-(4-phenylthioanilid) in 15 ml 1,2-Dimethoxiethan zu und rührte 30 min bei 24° und 3 Stdn. unter Rückflußkochen. Nach dem Abkühlen wurde abgesaugt und der Filterrückstand mit Wasser und Aceton ausgewaschen. Der nach dem Eindampfen des Filtrats verbliebene Rückstand wurde in Wasser suspendiert, abgesaugt und mit Wasser ausgewaschen. Beide Feststoffanteile wurden mit einer Lösung von 2,5 g $NaHCO_3$ in 100 ml Wasser versetzt und 4 Stdn. geschüttelt. Nach dem Absaugen wusch man den Feststoff mit Wasser und kristallisierte ihn aus Methanol/Dimethylformamid 1,0 : 1 um. Zusammen mist einem nach Eindampfen der Mutterlauge und erneuter Umkristallisation des dabei verbliebenen Rückstands aux Methanol erhaltenen zweiten Kristallisat wurden auf diese Weise 3,65 g (79 %) bezogen auf 10 mMol) reines 4-Amino-2-(4,4-diethyl-imidazolidin-2-on-1-yl)pyrimidin-5-carbonsäure-(4-phenylthio-anilid) vom Fp. 265-6 °C ;

(IR : (Ureido) CO : 5,81 μ) erhalten.

Analyse : $C_{24}H_{26}N_6O_2S$
ber. (%) : C 62,3 ; H 5,7 ; N 18,2 ; S 6,9 MG 462,6
gef. (%) : C 62,1 ; H 5,8 ; N 17,8 ; S 6,7

Beispiel 33

Nach der gleichen Arbeitsweise wie im Beispiel 27 beschrieben wurde ausgehend von 6,9 g (26 mMol) rohem, öligen 1-Amidino-4-methyl-4-propyl-imidazolidin-2-on-hydrobromid und 6,0 g (21 mMol) Ethoximethylencyanacet-(3-trifluormethyl-anilid) 6,6 g (79 % bezogen auf 21 mMol) reines 4-Amino-2-(4-methyl-4-propyl-imidazolidin-2-on-1-yl)-pyrimidin-5-carbonsäure-(3-trifluormethyl-anilid) vom Fp. 248-9 °C erhalten (das Rohprodukt war aus Aceton umkristallisiert worden).

Analyse : $C_{19}H_{21}F_3N_6O_2$
ber. (%) : C 54,0 ; H 5,0 ; F 13,5 ; N 19,9 MG 422,4
gef. (%) : C 53,8 ; H 5,3 ; F 13,5 ; N 19,6 MG 422,0 (massenspektroskopisch)

Beispiel 34

a) 4-Amino-2-(4,4-dimethyl-imidazolidin-2-on-1-yl)-pyrimidin-5-carbonsäure-(3-chlor-2-methyl-anilid)-hydrochlorid :

Zu einer Suspension von 7,50 g (20 mMol) 4-Amino-2-(4,4-dimethyl-imidazolidin-2-on-1-yl)-pyrimidin-5-carb-(3-chlor-2-methyl-anilid) in 120 ml abs. Ethanol tropfte man unter Kühlung 3,24 ml einer 6,3 molaren Lösung von HCl in Ether und schüttelte dann den entstandenen Brei 1 Std. bei Raumtemperatur. Anschließend saugte man ab, wusch mit Ethanol und Ether nach und trocknete das Hydrochlorid. Man erhielt 8,15 g des oben genannten Hydrochlorids vom Fp. 300-302 °C.

Analyse : $C_{17}H_{20}CL_2N_6O_2$
ber. (%) : C 49,6 ; H 4,9 ; Cl 17,2 ; N 20,4
gef. (%) : C 49,3 ; H 5,1 ; Cl 17,1 ; N 20,0 ; $Cl^{\ominus}$ 8,6

b) Analog dieser Arbeitsweise wurde 4-Amino-2-(4,4-dimethyl-imidazolidin-2-on-1-yl)-pyrimidin-5-carbonsäure-(5-chlor-2-methyl-anilid)-hydrochlorid hergestellt ; Fp. 293-5 °C.

Analyse : $C_{17}H_{20}Cl_2N_6O_2$
gef. (%) : C 49,3 ; H 5,1 ; Cl 17,2 ; N 20,0 ; $Cl^{\ominus}$ 8,8

c) Analog der unter a) beschriebenen Arbeitsweise wurde 4-Amino-2-(4,4-dimethyl-imidazolidin-2-on-1-yl)-pyrimidin-5-carbonsäure-(3-trifluormethyl-anilid)-hydrochlorid hergestellt ; Fp. 318-9 °C,

Analyse : $C_{17}H_{18}ClF_3N_6O_2$
ber. (%) : C 47,4 ; H 4,2 ; N 19,5 ; $Cl^{\ominus}$ 8,2
gef. (%) : C 47,2 ; H 4,2 ; N 19,2 ; $Cl^{\ominus}$ 8,0

Beispiel 35

4-Amino-2-(4,4-dimethyl-imidazolidin-2-on-1-yl)-pyrimidin-5-carbonsäure-(3-trifluormethyl-anilid)-p-toluolsulfonat :

Zu einer heißen Suspension von 1,97 g (5 mMol) 4-Amino-2-(4,4-dimethylimidazolidin-2-on-1-yl)-pyrimidin-5-carb-(3-trifluormethyl-anilid) in 40 ml abs. Ethanol gab man eine heiße Lösung von 0,95 g (5 mMol) p-Toluolsulfonsäurehydrat in 5 ml abs. Ethanol, wonach kurzzeitig eine Lösung entstand, die 5 min unter Rückfluß gekocht wurde. Nach dem Kühlen saugte man das ausgeschiedene Kristallisat ab und wusch es mit Ethanol und Ether aus. Nach dem Trocknen wurden 2,52 g (≙ 86,3 % Ausbeute) des oben genannten p-Toluolsulfonats vom Fp. 223-4 °C erhalten.

Analyse : $C_{24}H_{25}F_3N_6O_5S$
ber. (%) : C 50,9 ; H 4,4 ; F 10,1 ; N 14,8 ; S 5,7
gef. (%) : C 50,5 ; H 4,5 ; F 9,8 ; N 14,5 ; S 5,9

Beispiel 36

Zu einer Lösung von 1,15 g Natrium in 60 ml Isopropanol gab man 5,0 g (52 mMol) Guanidiniumchlorid, kochte 20 min unter Rückfluß und saugte nach dem Abkühlen vom ausgeschiedenen NaCl ab. Zum Filtrat tropfte man bei − 10 °C in 15 min eine Lösung von 5,0 g (50 mMol) Butylisocyanat in 20 ml 1,2-Dimethoxiethan, rührte je 30 min bei 0 und 25 °C und 1 Std. bei 40 °C nach. kühlte auf Raumtemperatur ab und setzte 13,2 g (50 mMol) Ethoximethylen-cyanacet-(4-chlor-2-methyl-anilid) zu und rührte die Suspension 20 min bei Raumtemperatur und 2 Stdn. unter Rückflußkochen. Nach dem Abkühlen wurde der Feststoff abgesaugt und mit Aceton ausgewaschen. Man erhielt so 8,2 g kristallines Rohprodukt. Durch Einengen des Filtrats wurden weitere 3,6 g dieses Stoffes erhalten. Der Feststoff wurde aus

Acetonitril/Dimethylformamid 4 : 1 umkristallisiert, wobei 10,84 g (58 %) reines 4-Amino-2-(3-butyl-1-ureido)-pyrimidin-5-carbonsaüre-(4-chlor-2-methyl-anilid) vom Fp. 263-4 °C erhalten wurden.

Analyse : $C_{17}H_{21}ClN_6O_2$.
ber. (%) : C 54,2 ; H 5,6 ; Cl 9,4 ; N 22,3 ; MG 376,9
gef. (%) : C 53,8 ; H 5,5 ; Cl 9,4 ; N 22,4 ; MG 376 ; 378 (massenspektroskopisch)

Beispiel 37

Zu einer Lösung von 0,69 g Natrium in 40 ml Isopropanol gab man 3,0 g (31 m Mol) Guanidiniumchlorid, kochte 20 min unter Rückfluß und saugte nach dem Abkühlen bei ca. 10 °C vom ausgefallenen NaCl ab. Zum Filtrat tropfte man bei – 10 °C in 25 min eine Lösung von 4,2 g (33,5 mMol) Cyclohexylisocyanat in 20 ml abs. Dimethoxiethan, rührte je 30 min von – 10 °C bis Raumtemperatur und bei Raumtemperatur und 1 Std. bei 40 °C nach, kühlte auf Raumtemperatur ab, setzte 8,55 g (30 mMol) Ethoximethylencyanacet-(3-trifluormethyl-anilid) zu und rührte die Mischung 20 min bei Raumteperatur und 2,5 Stdn. unter Rückflußkochen. Beim Abkühlen fiel kristallines Produkt aus, das abgesaugt und mit Aceton ausgewaschen wurde. Man erhielt auf diese Weise 5,2 g (41 %) reines 4-Amino-2-(3-cyclohexyl-1-ureido)-pyrimidin-5-carbonsäure-(3-trifluormethyl-anilid) vom Fp. 268-9 °C.

Analyse : $C_{19}H_{21}F_3N_6O_2$.
ber. (%) : C 54,0 ; H 5,0 ; F 13,5 ; N 19,9 ; MG 422,4
gef. (%) : C 53,8 ; H 5,1 ; F 13,2 ; N 19,8 ; MG 422,0 (massenspektroskopisch)

Analog der in den Beispielen 36 und 37 beschriebenen Arbeitsweise wurden die in der Tabelle 3 (Beispiele 38-54) aufgeführten 4-Amino-2-ureido-pyrimidin-5-carbanilide hergestellt. Die Isolierung des jeweiligen Endprodukts erfolgte teils gemäß der im Beispiel 36 beschriebenen Arbeitsweise, d.h. weitere Anteile an Rohprodukt wurden durch Einengen bzw. Eindampfen des Filtrats erhalten und das gesamte Rohprodukt wurde aus Dimethylformamid/Acetonitril umkristallisiert ; bei einem Teil der Beispiele fiel ähnlich wie im Beispiel 37 das Endprodukt direkt in reiner Form an und aus dem eingeengten Filtrat wurde keine weitere Menge dieses Produktes mehr erhalten. Die Ausbeuten der in den Beispielen 38-54 (Tab. 3) erhaltenen Verbindungen lagen zwischen 39 und 63 %.

Tabelle 3*
Beispiele 38-54

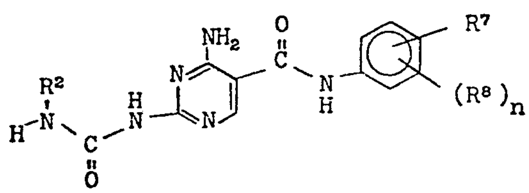

| Beispiel Nr. | $R^2$ | $R^7$ | $R^8$ | n | Fp. °C** |
|---|---|---|---|---|---|
| 38 | $CH_3$ | H | — | 0 | 252-4 |
| 39 | $C_4H_9$ | $4—CH(CH_3)_2$ | 3—Cl | 1 | 252-3 |
| 40 | $C_4H_9$ | $4—OC_2H_5$ | 3—Cl | 1 | 259-60 |
| 41 | $C_4H_9$ | $4—OC_6H_5$ | 3—Cl<br>$6—CH_3$ | 2 | 255-6 |
| 42 | tert.—$C_4H_9$ | 3—Cl | $2—CH_3$ | 1 | 247-8 |
| 43 | tert.—$C_4H_9$ | $4—OC_2H_5$ | 3—Cl | 1 | 246-8 |
| 44 | tert.—$C_4H_9$ | $3—CF_3$ | — | 0 | 261-2 |
| 45 | 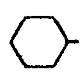 | 3—Cl | $2—CH_3$ | 1 | 252-3 |
| 46 | $C_8H_{17}$ | 3—Cl | $2—CH_3$ | 1 | 263-4 |
| 47 | $C_8H_{17}$ | $4—OC_2H_5$ | 3—Cl | 1 | 264-5 |
| 48 | 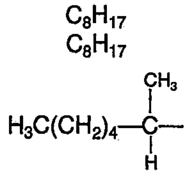 | $3—CF_3$ | — | 0 | 259-60 |

Tabelle 3 (Fortsetzung)

| Beispiel Nr. | $R^2$ | $R^7$ | $R^8$ | n | Fp. °C** |
|---|---|---|---|---|---|
| 49 | $C_6H_5$ | 4—$OCH_3$ | — | 0 | 277-9 |
| 50 | 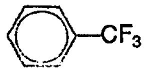 | 4—Cl | 3—$CH_3$ | 1 | 281-3 |
| 51 | 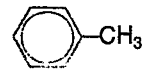 | 4—$OC_2H_5$ | 3—Cl | 1 | 279-81 |
| 52 | 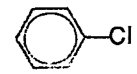 | 4—$OC_6H_5$ | H | 0 | 282-3 |
| 53 | $C_6H_5$ | 3—Cl | 2—$CH_3$ | 1 | 298-300 |
| 54 | $C_4H_9$ | 3—Cl | 2—$CH_3$ | 1 | 250-1 |

*) Die gemäß den Beispielen 38-54 erhaltenen Verbindungen ergaben die den Bruttoformeln entsprechenden korrekten Analysenwerte und Molgewichte (massenspektroskopisch ermittelt).
**) Bei den Schmelzpunkten handelt es sich um Zersetzungspunkte

## Beispiel 55

a) 2,4-Diamino-pyrimidin-5-carbonsäure-(3-chlor-2-methyl-anilid) :

Zu einer Lösung von 2,3 g Natrium in 160 ml Isopropanol gab man 10 g (105 mMol) Guanidiniumchlorid, kochte 20 min unter Rückfluß und saugte nach dem Abkühlen vom ausgeschiedenen NaCl ab. Zum Filtrat gab man eine Suspension von 26,5 g (0,1 Mol) Ethoximethylen-cyanacet-(3-chlor-2-methyl-anilid) in 100 ml Dioxan und rührte je 20 min bei Raumtemperatur und bei 60 °C und 1,5 Stdn. unter Rückflußkochen.

Man kühlte die Mischung, saugte den Feststoff ab und wusch diesen mit Methanol aus. Nach Einengen des Filtrats schied sich weiterer Feststoff ab, der auf die gleiche Weise isoliert wurde. Man erhielt insgesamt 23,6 g Rohprodukt, das aus Acetonitril/Dimethylformamid 3 : 1 umkristallisiert, einschließlich der Aufarbeitung der eingeengten Mutterlauge, 20,3 g (73 %) reines 2,4-Diamino-pyrimidin-5-carbonsäure-(3-chlor-2-methyl-anilid) vom Fp. 291-2 °C ergab.

Analyse : $C_{12}H_{12}ClN_5O$
ber. (%) : C 51,9 ; H 4,4 ; Cl 12,8 ; N 25,2 ; MG 277,7
gef. (%) : C 51,8 ; H 4,4 ; Cl 13,1 ; N 25,1 ; MG 277 ; 279 (massenspektroskopisch)

b) 4-Amino-2-(3-butyl-1-ureido)-pyrimidin-5-carbonsäure-(3-chlor-2-methyl-anilid) :

Eine Mischung aus 2,78 g (10 mMol) 2,4-Diamino-pyrimidin-5-carb-(3-chlor-2-methyl-anilid), 35 ml trocknem Dimethylformamid, 1,40 g (14 mMol) Butylisocyanat und 0,40 g (4 mMol) Triethylamin wurde unter Rühren 16 Stdn. auf 85 °C erwärmt und anschließend im Vakuum eingedampft. Den Rückstand kochte man je 1 Std. zweimal mit je 50 ml Methanol aus und saugte jeweils im kalten Zustand den ungelöst verbliebenen Feststoff ab. Dieser wurde aus Acetonitril/Dimethylformamid 5:1 umkristallisiert, wobei 1,38 g (37 %) reines 4-Amino-2-(3-butyl-1-ureido)-pyrimidin-5-carb-(3-chlor-2-methyl-anilid) vom Fp. 249-51 °C erhalten wurden. Diese Verbindung erwies sich laut IR-Spektrum, Schmelzpunkt und Mischschmelzpunkt als identisch mit der gemäß Beispiel 54 (Tab. 2) auf anderem Weg erhaltenen.

Gemäß der unter a) beschriebenen Arbeitsweise wurden folgende 2,4-Diamino-pyrimidin-5-carbanilide hergestellt :

2,4-Diamino-pyrimidin-5-carb-(4-chlor-3-methyl-anilid) ; Fp. 246-47 °C ; Analyse : gef. (%) : C 51,6 H 4,3 Cl 12,7 N 24,8 MG 277 ; 279 (massenspektroskopisch)

~-(3-chlor-4-ethoxi-anilid), Fp. 245-6 °C
Analyse : $C_{13}H_{14}ClN_5O_2$ :
gef. (%) : C 50,8 ; H 4,5 ; Cl 12,1 ; N 22,8 MG 307 ; 309 (massenspektroskopisch)
ber. (%) : C 50,7 ; H 4,6 ; Cl 11,5 ; N 22,8 MG 307,7

~-(3-trifluormethyl-anilid), Fp. 223-4 °C
Analyse : $C_{12}H_{10}F_3N_5O$ :

ber. (%) : C 48,5 ; H 3,4 ; F 19,2 ; N 23,6 ; MG 297,3
gef. (%) : C 48,6 ; H 3,4 ; F 19,3 ; N 23,4 ; MG 297 (massenspektroskopisch)

Gemäß der unter b) beschriebenen Arbeitsweise wurden folgende 4-Amino-2-ureido-pyrimidin-5-carbanilide hergestellt :
4-Amino-2-(3-phenyl-1-ureido)-pyrimidin-5-carb-(3-chlor-2-methyl-anilid) ; Fp. 298-300 °C (Zers.) (Ausbeute 16 %).

Analyse : $C_{19}H_{17}ClN_6O_2$
ber. (%) : C 57,5 ; H 4,3 ; Cl 8,9 ; N 21,2
gef. (%) : C 57,0 ; H 4,1 ; Cl 8,5 ; N 21,5

4-Amino-2-(3-(3,4-methylendioxi-phenyl)-1-ureido)-pyrimidin-5-carb-(4-chlor-5-methyl-anilid) ; Fp. 276-7 °C (aus DMF/Acetronitril) (Ausbeute 18 %).

Analyse : $C_{20}H_{17}ClN_6O_5$
ber. (%) : C 54,5 ; H 3,9 ; Cl 8,0 ; N 19,1 ; MG 440,9
gef. (%) : C 53,9 ; H 3,9 ; Cl 7,9 ; N 18,8 ; MG 440 ; 442 (massenspektroskopisch)

## Beispiel 56

Zu einer Lösung von 0,69 g Natrium in 40 ml Isopropanol gab man 3,0 g (31 mMol) Guanidiniumchlorid, kochte 15 min unter Rückfluß und saugte nach dem Abkühlen vom ausgeschiedenen NaCl ab. Das Filtrat wurde im Vakuum auf etwa die Hälfte des Volumens eingeengt. Dann tropfte man zu dieser eingeengten Lösung bei − 10 °C in 25 min eine Lösung von 4,9 (30 mMol) 3,4-Methylendioxiphenylisocyanat in 30 ml abs. Dimethoxiethan, rührte 20 min bei − 10 bis 24 °C und je 1 Std. bei 24 und bei 40-42 °C. Nach dem Abkühlen wurde der ausgefallene Feststoff abgesaugt und das Filtrat im Vakuum bei 40 °C Badtemperatur eingedampft. Den Rückstand nahm man in 30 ml Ethylacetat auf. Aus der Lösung kristallisierte langsam weiterer (3,4-Methylendioxi-phenyl)-guanylharstoff aus. Insgesamt wurden von dieser Verbindung 4,1 g (62 % d.Th.) (Fp. ab 250 °C Verkohlung) erhalten. Dieses Produkt wurde in 50 ml Dimethoxiethan und 25 ml Isopropanol suspendiert und mit 5,1 g (19 mMol) Ethoximethylencyanacet-(4-chlor-3-methyl-anilid) versetzt. Diese Mischung rührte man 20 min bei 23 °C und 2,5 Stdn. unter Rückflußkochen. Nach dem Abkühlen saugte man den Feststoff ab, wusch diesen mit Aceton und kristallisierte ihn aus Dimethylformamid/Acetonitril 2 : 1 um. Man erhielt dann nach dem Trocknen 2,10 g (26 % bezogen auf 18,5 mMol) reines 4-Amino-2-(3-(3,4-methylendioxi-phenyl)-1-ureido)-pyrimidin-5-carb-(4-chlor-3-methyl-anilid) vom Fp. 276-8 °C.

Analyse : $C_{20}H_{17}ClN_6O_4$
gef. (%) : C 54,0 ; H 3,8 ; Cl 8,0 ; N 18,7
ber. (%) : C 54,5 ; H 3,9 ; Cl 8,0 ; N 19,1

## Beispiel 57

Gemäß der im Beispiel 56 beschriebenen Arbeitsweise wurde 4-Amino-2-(3-(3,4-methylendioxi-phenyl)-1-ureido)-pyrimidin-5-carb-(3-trifluormethyl-anilid) vom Fp. 240-2 °C erhalten (Ausbeute 19 %)

Analyse : $C_{20}H_{15}F_3N_6O_4$
ber. (%) : C 52,2 ; H 3,3 ; F 18,3 ; MG 460,4
gef. (%) : C 51,8 ; H 3,2 ; F 11,8 ; MG 460,0 (massenspektroskopisch)

## Beispiel 58

4-Amino-2-(3-oktyl-1-ureido)-pyrimidin-5-carbonsäure-(3-chlor-2-methyl-anilid)-hydrochlorid :

Zu einer Suspension von 2,165 g (5 mMol) 4-Amino-2-(3-oktyl-1-ureido)-pyrimidin-5-carb-(3-chlor-2-methyl-anilid) in 15 ml abs. Ethanol gab man 0,81 ml einer 6,3 molaren Lösung von HCl in Ether und rührte 1 Std. bei Raumtemperatur. Danach saugte man den Feststoff ab und wusch ihn mit Ethanol und Ether nach. Das so erhaltene Hydrochlorid wog nach dem Trocknen 2,28 g und schmolz bei 220-2 °C, verfestigte sich wieder und schmolz danach bei 323-5 °C.

Analyse : $C_{21}H_{30}Cl_2N_6O_2$
ber. (%) : C 53,7 ; H 6,4 ; Cl 15,1 ; N 17,9
gef. (%) : C 53,3 ; H 6,3 ; Cl 15,0 ; N 18,0 $Cl^{\ominus}$ 7,4

Beispiel 59

Zu einer Lösung von 0,92 g Natrium in 55 ml Isopropanol gab man 4,0 g (42 mMol) Guanidinium chlorid, kochte 15 min unter Rückfluß und saugte bei 10 °C vom ausgeschiedenen NaCl ab. Zum Filtrat tropfte man bei – 10 °C in 20 min eine Lösung von 4 g (40 mMol) Butylisocyanat in 40 ml Dimethoxiethan, rührte je 30 min bei 0 °C und bei Raumtemperatur und 1 Std. bei 40 °C nach, setzte dann zu der auf Raumtemperatur abgekühlten Lösung von 11,3 g (ca. 40 mMol) rohem, öligen Ethoximethylen-cyanacet-(N-ethyl-4-chlor-anilid) in 80 ml Dimethoxiethan zu und rührte 40 min bei Raumtemperatur und 3 Stdn. unter Rückflußkochen nach. Anschließend wurde die Reaktionsmischung im Vakuum eingedampft. Der ölige Rückstand (18,4 g) wurde aus 60 ml $CH_2Cl_2$ auf eine 35 cm hohe (∅ = 5,0 cm) Kieselgel S (0,063-0,2 mm, Riedel-DeHäen AG)-$CH_2Cl_2$-Säule aufgezogen und chromatographiert. Nach Elution mit 800 ml $CH_2Cl_2$, je 300 ml $CH_2Cl/C_2H_5OH$ 100 : 2,600 ml $CH_2Cl_2/C_2H_5OH$ 100 : 3 und je 700 ml $CH_2Cl_2/C_2H_5OH$ 100 : 4 und 100 : 5 wurden mit je 600 ml $CH_2Cl_2/C_2H_5OH$ 100 : 5 und 100 : 6 2,8 g eines, laut IR-Spektrum eine Cyano-Gruppe enthaltenden Nebenproduktes und bei fortgesetzter Elution mit 300 ml $CH_2Cl_2/C_2H_5OH$ 100 : 7 und je 500 ml 100 : 8 und 100 : 9 1,80 g Produkt eluiert, in dem die erwünschte Verbindung angereichert war. Dieses Produkt ergab nach zweimaligen Umkristallisieren aus Methanol 0,92 g (6 %) reines 4-Amino-2-(3-butyl-1-ureido)-pyrimidin-5-carbonsäure-(N-ethyl-4-chlor-anilid) vom Fp. 267-9 °C.

Analyse : $C_{18}H_{23}ClN_6O_2$
ber. (%) : C 55,3 ; H 5,9 ; Cl 9,1 ; N 21,5 ; MG 390,9
gef. (%) : C 55,0 ; H 6,0 ; Cl 9,3 ; N 21,1 ; MG 390 ; 392 (massenspektroskopisch)

Beispiel 60

Zu einer Suspension von 5,95 g (20 mMol) 2,4-Diamino-pyrimidin-5-carbonsäure-(3-trifluormethyl-anilid) und 2,05 g Triethylamin in 100 ml Acetonitril tropfte man bei 75 °C in 20 min 3,30 g (2,83 ml, 31 mMol) N,N-Dimethylcarbamylchlorid und rührte 4 Stdn. bei 75 °C nach. Der suspendierte Feststoff wurde durch Filtrieren der 75 °C warmen Reaktionsmischung abgetrennt. Aus dem Filtrat fiel beim Kühlen auf 5 °C (20 Stdn.) kristallines Produkt aus. Dieses wurde abgesaugt, mit Acetonitril nachgewaschen und getrocknet. Auf diese Weise erhielt man 2,2 g (30 %) reines 4-Amino-2-(3,3-dimethyl-1-ureido)-pyrimidin-5-carbonsäure-(3-trifluormethyl-anilid) vom Fp. 238-40 °C (Fp. ist sehr stark von der Heizgeschwindigkeit abhängig, bei normalen Aufheizen gast das Produkt bei 236-8 °C auf und kristallisiert wieder ; obiger Fp. wurde erhalten, indem die Probe bei 210 °C in den Schmelzpunkt-Apparat (Büchi SMP-20) eingesetzt und dann mit 2 °C/min aufgeheizt wurde).

Analyse : $C_{15}H_{15}F_3N_6O_2$
ber. (%) : C 48,9 ; H 4,1 ; F 15,5 ; N 22,8 ; MG 368,3
gef. (%) : C 48,6 ; H 4,1 ; F 15,5 ; N 22,8 ; MG 368 (massenspektroskopisch)

Beispiel 61

Entsprechend der im Beispiel 60 beschriebenen Arbeitsweise wurden ausgehend von 5,95 g (20 mMol) 2,4-Diamino-pyrimidin-5-carb-(3-trifluormethyl-anilid) und 4,90 g (33 mMol) N,N-Pentamethylen-carbamylchlorid (vgl. Boon, J. Chem. Soc. 1947, 313) 1,55 g (19 %) reines 4-Amino-2-(3,3-pentamethylen-1-ureido)-pyrimidin-5-carbonsäure-(3-trifluormethyl-anilid) vom Fp. 228-231 °C (Zers.) (gleiches Verhalten am Zers.-Punkt wie bei der im Beispiel 60 beschriebenen Verbindung) erhalten.

Analyse : $C_{18}H_{19}F_3N_6O_2$
ber. (%) : C 52,9 ; H 4,7 ; F 14,0 ; N 20,6 ; MG 408,4
gef. (%) : C 52,6 ; H 4,6 ; F 13,6 ; N 20,4 ; MG 408 (massenspektroskopisch)

Beispiel 62

Gemäß der im Beispiel 4 beschriebenen Arbeitsweise wurden ausgehend von 5,0 g (20 mMol) Methoximethylen-cyanacet-(3-chlor-4-methyl-anilid) und 3,44 g (22 mMol) 1-Amidino-4,4-dimethyl-imidazolidin-2-on 6,83 g (91 %) reines 4-Amino-2-(4,4-dimethyl-imidazolidin-2-on-1-yl)-pyrimidin-5-din-(3-chlor-4-methyl-anilid) vom Fp. 290-2 °C erhalten. Das Produkt war identisch (laut IR-Spektrum und Fp.) mit der nach Beispiel 4 erhaltenen Verbindung.

Beispiel 63

Zu einer Suspension von 3,42 g (12 mMol) 1-Amidino-4-phenyl-imidazolidin-2-on-hydrobromid in 25 ml abs. Ethanol tropfte man bei 0 °C eine Lösung von 0,285 g (12 mg Atom) Natrium in 20 ml abs. Ethanol, rührte 15 min bei 23 °C, gab dann eine Suspension von 3,80 g (13,3 mMol) Ethoximethylen-cyanacet-(3-trifluormethyl-anilid) in 25 ml 1,2-Dimethoxiethan zu und rührte 20 min bei Raumtemperatur und 2 Stdn. unter Rückflußkochen. Nach dem Abkühlen wurde abgesaugt und der Filterrückstand mit Wasser und Ethanol nachgewaschen. Das Filtrat dampfte man im Vakuum ein und den verbleibenden Rückstand vermischte man mit Ethylacetat, wobei weiteres kristallines Material anfiel, das ebenfalls durch Absaugen isoliert wurde. Die beiden isolierten kristallinen Stoffe (zusammen 6,0 g) wurden vereinigt, mit einer Lösung von 2 g $NaHCO_3$ in 100 ml Wasser versetzt und 4 Stdn. geschüttelt. Nach dem Absaugen wusch man den Feststoff mit Wasser und kristallisierte ihn aus Methanol um. Zusammen mit einem nach Eindampfen der Mutterlauge und erneuter Umkristallisation des dabei angefallenen Rückstands aus Methanol erhaltenen zweiten Kristallisat wurden auf diese Weise (nach Trocknung) 4,50 g (85 %) reines 4-Amino-2-(4-phenyl-imidazolidin-2-on-1-yl)-pyrimidin-5-carbonsäure-(3-trifluormethyl-anilid) vom Fp. 172-4 °C erhalten.

Analyse : $C_{21}H_{17}F_3N_6O_2$

| | | | | | | |
|---|---|---|---|---|---|---|
| ber. (%) : | C 57,0 ; | H 3,9 ; | F 12,9 ; | N 19,0 ; | MG 442,4 | |
| gef. (%) : | C 56,8 ; | H 4,1 ; | F 12,5 ; | N 18,7 ; | MG 442 | (massenspektroskopisch) |

Beispiele 64-74

Entsprechend der im Beispiel 4 beschriebenen Arbeitsweise wurden die in der folgenden Tabelle 4 aufgeführten 4-Amino-2-(imidazolidin-2-on-1-yl)-pyrimidin-5-carbanilide hergestellt. Die als Ausgangsstoffe dafür nötigen Ethoximethylencyanacetanilide sind in der Tabelle 5 zusammengestellt.

Tabelle 4*

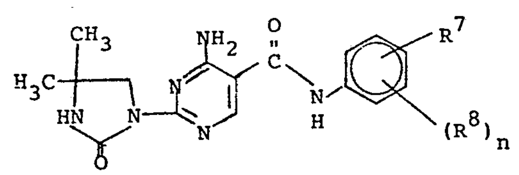

| Beisp. Nr. | $R^7$ | $R^8$ | n | Fp. °C** | Ausb. in % |
|---|---|---|---|---|---|
| 64 | 2—$CF_3$ | — | 0 | 265-6 | 87 |
| 65 | 4—$CF_3$ | — | 0 | 320-1 | 97 |
| 66 | 2—$CF_3$ | 4—Cl | 1 | 275-6 | 89 |
| 67 | 5—$CF_3$ | 2—F | 1 | 274-5 | 93 |
| 68 | 5—$CF_3$ | 2—Cl | 1 | 262-3 | 94 |
| 69 | 3—$CF_3$ | 4—Cl | 1 | 298-9 | 92 |
| 70 | 4—$OC_6H_5$ | 3—$CF_3$ | 1 | 240-1 | 97 |
| 71 | 4—$SC_6H_5$ | 3—$CF_3$ | 1 | 282-3 | 98 |
| 72 | 3—$CF_3$ | 5—$CF_3$ | 1 | 304-5 | 82 |
| 73 | 3—$CF_3$ | 4—$OCH_3$ | 1 | 301-2 | 95 |
| 74 | 4—O— 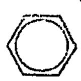 —$OCH_3$ | 3—$CF_3$ | 1 | 263-4 | 82 |

*) Die gemäß den Beispielen 64-74 erhaltenen Verbindungen ergaben die den Bruttoformeln entsprechenden korrekten Analysenwerte. Im IR-Spektrum enthalten diese Verbindungen eine charakteristische, intensive Bande des Imidazolidinon-Carbonyls bei 5.76-5.82 μ.

**) Bei den Schmelzpunkten handelt es sich in der Mehrzahl der Fälle um Zersetzungspunkte.

Tabelle 5
Ethoximethylen-cyanacetanilide

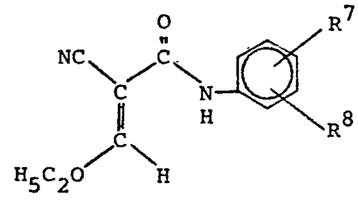

| IIIA | $R^7$ | $R^8$ | Fp. °C | Bemerkungen |
|---|---|---|---|---|
| a | 2—$CF_3$ | — | 90-1 | E—Form |
| b | 4—$CF_3$ | — | 189-91 | E—Form |
| c | 2—$CF_3$ | 4—Cl | 107-8 | E—Z-Mischung |
| d | 5—$CF_3$ | 2—F | 167-8 | Z—Form |
| e | 5—$CF_3$ | 2—Cl | 165-6 | E—Z—Mischung |
| f | 3—$CF_3$ | 4—Cl | 187-8 | E—Form |
| g | 3—$CF_3$ | 5—$CF_3$ | 156-7 | E—Form |
| h | 4—$OC_6H_5$ | 3—$CF_3$ | 136-7 | E—Form |
| i | 4—$SC_6H_5$ | 3—$CF_3$ | 160-1 | E—Form |
| k | 3—$CF_3$ | 4—$OCH_3$ | 188-9 | E—Form |
| l | 4—O— 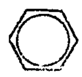 —$OCH_3$ | 3—$CF_3$ | 151-2 | E—Form |

Beispiele 75-81

Entsprechend der im Beispiel 27 beschriebenen Arbeitsweise wurden die in der folgenden Tabelle 6 aufgeführten 4-Amino-2-(imidazolidin-2-on-1-yl)-pyrimidin-5-carbanilide hergestellt. Die als Ausgangsstoffe dafür nötigen Ethoximethylencyanacetanilide sind in der Tabelle 5 zusammengestellt.

Tabelle 6*

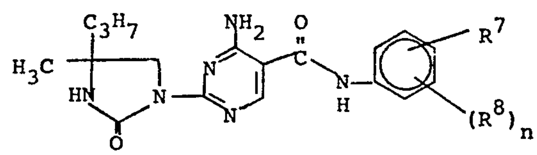

| Beisp. Nr. | $R^7$ | $R^8$ | n | Fp. °C** | Ausb. in % |
|---|---|---|---|---|---|
| 75 | 2—$CF_3$ | — | 0 | 258-9 | 86 |
| 76 | 4—$CF_3$ | — | 0 | 272-3 | 90 |
| 77 | 5—$CF_3$ | 2—F | 1 | 239-40 | 93 |
| 78 | 5—$CF_3$ | 2—Cl | 1 | 245-6 | 96 |
| 79 | 3—$CF_3$ | 4—Cl | 1 | 288-9 | 93 |
| 80 | 4—$OC_6H_5$ | 3—$CF_3$ | 1 | 256-7 | 86 |
| 81 | 4—$SC_6H_5$ | 3—$CF_3$ | 1 | | |

*) Die gemäß den Beispielen 75-81 erhaltenen Verbindungen ergaben die den Bruttoformeln entsprechenden korrekten Analysenwerte. Im IR-Spektrum enthalten diese Verbindungen eine charakteristische, intensive Bande des Imidazolidinon-Carbonyls bei 5.76-5.82 μ.

**) Bei den Schmelzpunkten handelt es sich in der Mehrzahl der Fälle um Zersetzungspunkte.

**Ansprüche** (für die Vertragsstaaten : BE, CH, DE, FR, GB, IT, NL, SE)

1. 4-Amino-2-ureido-(bzw. -thioureido)-pyrimidin-5-carbonsäureanilide der allgemeinen Formel I

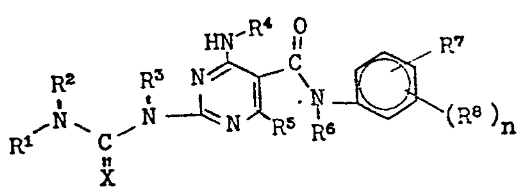

(I)

in der

$R^1$ und $R^3$, die gleich oder verschieden sind, Wasserstoff, eine $(C_1\text{-}C_3)$-Alkylgruppe oder einen Phenylrest,

$R^2$ Wasserstoff, eine $(C_1\text{-}C_{10})$-Alkylgruppe, eine $(C_3\text{-}C_4)$-Alkenylgruppe, eine $(C_4\text{-}C_8)$-Cycloalkylgruppe, eine Benzylgruppe oder eine Phenylgruppe, worin die Phenylreste zusätzlich durch 1-2 $(C_1\text{-}C_3)$-Alkylgruppen, 1-2 Halogenatome, 1-2 $(C_1\text{-}C_4)$-Alkoxigruppen, eine Trifluormethylgruppe, eine Methoxi- oder Ethoxicarbonylgruppe und/oder eine Methylendioxigruppe substituiert sein können,

$R^4$ Wasserstoff oder eine $(C_1\text{-}C_8)$-Acylgruppe,

$R^5$ Wasserstoff

$R^6$ Wasserstoff, eine $(C_1\text{-}C_3)$-Alkylgruppe, eine $(C_3\text{-}C_4)$-Alkenylgruppe, eine, gegebenenfalls durch 1 oder 2 Chloratome oder 1 oder 2 Methoxigruppen im Phenylrest substituierte, Benzylgruppe oder einen, gegebenenfalls durch 1 Methylgruppe und/oder 1 Chloratom substituierten, Phenylrest,

$R^7$ Wasserstoff, eine $(C_1\text{-}C_3)$-Alkylgruppe, eine $(C_3\text{-}C_4)$-Alkenylgruppe, eine $(C_5\text{-}C_6)$-Cycloalkylgruppe, eine Phenylgruppe, ein Halogenatom, eine Trifluormethyl-, $(C_1\text{-}C_4)$-Alkylthio-, $(C_1\text{-}C_2)$-Alkoxicarbonyl-, Cyano-, Acetamino-, Amino-, Nitro-, Carboxyl- oder $(C_1\text{-}C_4)$-Alkoxigruppe oder einen Rest

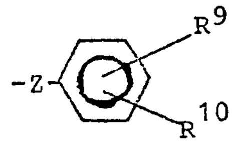

worin Z ein Sauerstoff- oder Schwefelatom oder eine $-\overset{O}{\overset{\|}{C}}-$, $-CH_2-$ oder $-CH_2CH_2-$Gruppe und $R^9$ und $R^{10}$, die gleich oder verschieden sind, ein Wasserstoff-, Fluor-, Chlor- oder Bromatom, eine $(C_1\text{-}C_3)$-Alkylgruppe, eine $(C_1\text{-}C_2)$-Alkoxigruppe, eine Acetamino- oder eine $(C_1\text{-}C_2)$-Alkoxicarbonylgruppe oder eine Carboxylgruppe bedeuten,

$R^8$ ein Fluor-, Chlor-, oder Bromatom oder eine Methyl-, $(C_1\text{-}C_2)$-Alkoxi- oder Trifluormethylgruppe

X ein Sauerstoff- oder Schwefelatom und

n = 0, 1, 2 oder 3 bedeuten, wobei die Reste

$R^1$ und $R^2$ zusammen auch einen verzweigten oder unverzweigten Alkylenrest mit 3-6 C-Atomen oder den $(-CH_2CH_2OCH_2CH_2-)$-Rest und

$R^2$ und $R^3$ zusammen einen verzweigten oder unverzweigten Alkylenrest mit 2-8 C-Atomen, der zusätzlich durch eine $(C_2\text{-}C_4)$-Alkenylgruppe oder einen, gegebenenfalls mit einem Chlor- oder Bromatom oder einer Methyl-, Ethyl-, Methoxi- oder Ethoxigruppe substituierten, Phenylrest substituiert sein kann, oder den $(-CH = CH-)$-Rest bedeuten, und wobei im Fall n = 1, die Reste $R^7$ und $R^8$ zusammen auch eine Methylen- oder Ethylendioxygruppe bedeuten können, sowie deren physiologisch verträgliche Säureadditionssalze.

2. 4-Amino-2-(4,4-dimethyl-imidazolidin-2-on-1-yl)-pyrimidin-5-carbonsäure-(3-trifluormethyl-anilid) gemäß Anspruch 1.

3. 4-Amino-2-(4,4-dimethyl-imidazolidin-2-on-1-yl)-pyrimidin-5-carbonsäure-(3-chlor-2-methyl-anilid) gemäß Anspruch 1.

4. 4-Amino-2-(4,4-dimethyl-imidazolidin-2-on-1-yl)-pyrimidin-5-carbonsäure-(2-fluor-5-trifluormethyl-anilid) gemäß Anspruch 1.

5. Verfahren zur Herstellung von Verbindungen gemäß Anspruch 1, dadurch gekennzeichnet, daß man

a) ein Isocyanat der allgemeinen Formel IIa

$$R^2 — N = C = O \qquad \text{(IIa)}$$

in der $R^2$ außer Wasserstoff die in Anspruch 1 angegebenen Bedeutungen hat, mit Guanidin umsetzt und die dabei gebildete Reaktionsmischung anschließend weiter mit einer Verbindung der allgemeinen Formel III

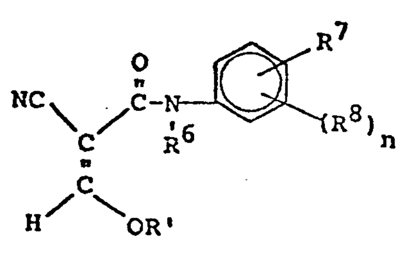

(III)

in der $R^6$, $R^7$ und $R^8$ sowie n die in Anspruch 1 bei der allgemeinen Formel I angegebenen Bedeutungen haben und R' eine Methyl- oder Ethylgruppe bedeutet, umsetzt oder

b) Verbindungen der allgemeinen Formel IV

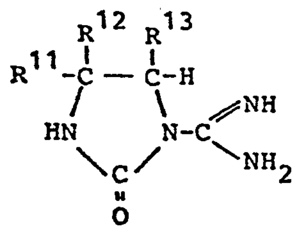

(IV)

in der $R^{11}$ Wasserstoff, $(C_1$-$C_3)$-Alkyl-, $(C_2$-$C_4)$-Alkenyl- oder, gegebenenfalls durch ein Chlor- oder Bromatom oder eine Methyl-, Ethyl, Methoxi- oder Ethoxigruppe substituiertes Phenyl, und $R^{12}$ und $R^{13}$, die gleich oder verschieden sein können, jeweils Wasserstoff oder eine $(C_1$-$C_3)$-Alkylgruppe bedeuten, oder deren Säureadditionssalze und für den Fall, daß Salze von Verbindungen der allgemeinen Formel IV verwendet werden, unter Zusatz einer basischen Verbindung mit einer Verbindung der allgemeinen Formel III, in der $R^6$-$R^8$, R' und n die oben angegebene Bedeutung haben, unter Bildung einer Verbindung der allgemeinen Formel I, in der X = 0 bedeutet und $R^1$, $R^4$ und $R^5$ Wasserstoff, und $R^2$ und $R^3$ zusammen eine

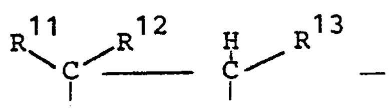

Gruppierung bedeuten, in der $R^{11}$-$R^{13}$ die obige Bedeutung haben, und $R^6$, $R^7$ und $R^8$ sowie n die oben genannte Bedeutung haben, umsetzt oder

c) ein Isocyanat der allgemeinen Formel IIa, oder ein Isothiocyanat der allgemeinen Formel IIb

$$R^2 — N = C = S \qquad \text{(IIb)}$$

in der $R^2$ außer Wasserstoff die in Anspruch 1 angegebene Bedeutung hat, mit einer Verbindung der allgemeinen Formel V

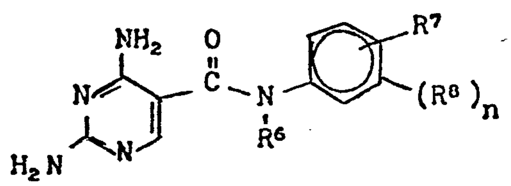

(V)

in der $R^6$, $R^7$ und $R^8$ sowie n die in Anspruch 1 bei der allgemeinen Formel I angegebenen Bedeutungen haben, umsetzt,

d) ein Chlorameisensäureamid bzw. ein Chlorthioameisensäureamid der allgemeinen Formel VI

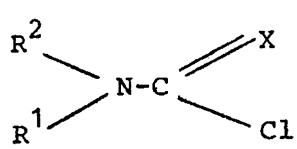

(VI)

in der $R^1$ eine $(C_1$-$C_3)$-Alkylgruppe oder einen Phenylrest, $R^2$ eine $(C_1$-$C_8)$-Alkylgruppe, eine $(C_3$-$C_4)$-Alkenylgruppe, eine $(C_5$-$C_6)$-Cycloalkylgruppe, eine Benzyl- oder Phenylgruppe, die gegebenenfalls im Phenylrest zusätzlich durch eine Methylgruppe, 1 oder 2 Chloratome, 1 oder 2 Methoxigruppen oder eine Methylendioxigruppe substituiert sein können, oder

$R^1$ und $R^2$ zusammen einen verzweigten oder unverzweigten Alkylenrest mit 3-6 C-Atomen oder den ($-CH_2CH_2OCH_2CH_2-$)-Rest und

X ein Sauerstoff- oder Schwefelatom bedeuten mit einer Verbindung der allgemeinen Formel V, in der $R^6$, $R^7$ und $R^8$ sowie n die in Anspruch 1 bei der allgemeinen Formel I angegebene Bedeutung haben, umsetzt, oder

e) 4-Amino-2-ureido-(bzw. 2-thioureido)-pyrimidin-5-carbonsäure-anilide der allgemeinen Formel I, in der $R^4$ Wasserstoff bedeutet und $R^1$, $R^2$, $R^3$, $R^5$, $R^6$, $R^7$, $R^8$, X und n die oben bei der allgemeinen Formel I angegebenen Bedeutungen haben, mit einem Acylierungsmittel der allgemeinen Formel VII

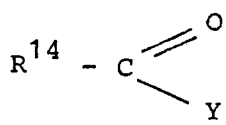

(VII)

in der

$R^{14}$ eine ($C_1$-$C_7$)-Alkylgruppe, einen Cyclohexyl- einen Benzyl- oder einen, gegebenenfalls durch eine Methylgruppe substituierten, Phenylrest und

Y ein Chlor- oder Bromatom oder die Gruppe $R^{14}-\overset{\overset{O}{\|}}{C}-O-$ bedeutet, umsetzt, und gegebenenfalls die nach Weg a)-e) erhaltenen Verbindungen der allgemeinen Formel I mit anorganischen oder organischen Säuren in ihre physiologisch verträglichen Säureadditionssalze überführt.

6. Arzneimittel auf Basis einer Verbindung gemäß Anspruch 1.

**Ansprüche** (für den Vertragsstaat AT)

1. Verfahren zur Herstellung von 4-Amino-2-ureido-(bzw. thioureido)-pyrimidin-5-carbonsäureanilide der allgemeinen Formel I

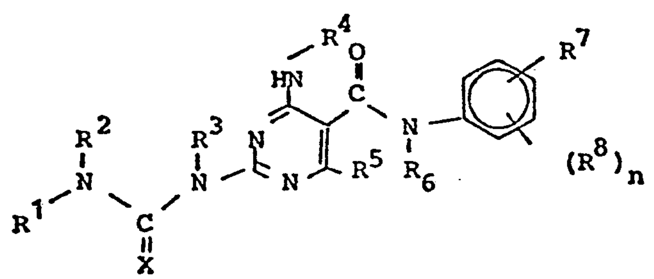

(I)

in der

$R^1$ und $R^3$, die gleich oder verschieden sind, Wasserstoff, eine ($C_1$-$C_3$-Alkylgruppe oder einen Phenylrest,

$R^2$ Wasserstoff, eine ($C_1$-$C_{10}$)-Alkylgruppe, eine ($C_3$-$C_4$)-Alkenylgruppe, eine ($C_4$-$C_8$)-Cycloalkylgruppe, eine Benzylgruppe oder eine Phenylgruppe, worin die Phenylreste zusätzlich durch 1-2 ($C_1$-$C_3$)-Alkylgruppen, 1-2 Halogenatome, 1-2 ($C_1$-$C_4$)-Alkoxigruppen, eine Trifluormethylgruppe, eine Methoxi- oder Ethoxicarbonylgruppe und/oder eine Methylendioxigruppe substituiert sein können,

$R^4$ Wasserstoff oder eine ($C_1$-$C_8$)-Acylgruppe,

$R^5$ Wasserstoff

$R^6$ Wasserstoff, eine ($C_1$-$C_3$)-Alkylgruppe, eine ($C_3$-$C_4$)-Alkenylgruppe, eine, gegebenenfalls durch 1 oder 2 Chloratome oder 1 oder 2 Methoxigruppen im Phenylrest substituierte, Benzylgruppe oder einen, gegebenenfalls durch 1 Methylgruppe und/oder 1 Chloratom substituierten, Phenylrest,

$R^7$ Wasserstoff, eine ($C_1$-$C_3$)-Alkylgruppe, eine ($C_3$-$C_4$)-Alkenylgruppe, eine ($C_5$-$C_6$)-Cycloalkylgruppe, eine Phenylgruppe, ein Halogenatom, eine Trifluormethyl-, ($C_1$-$C_4$)-Alkylthio-, ($C_1$-$C_2$)-Alkoxicarbonyl-, Cyano-, Acetamino-, Amino-, Nitro-, Carboxyl- oder ($C_1$-$C_4$)-Alkoxigruppe oder einen Rest

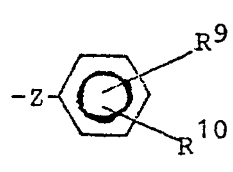

worin Z ein Sauerstoff- oder Schwefelatom oder eine $-\overset{O}{\overset{\|}{C}}-$, $-CH_2-$ oder $-CH_2CH_2$-Gruppe und $R^9$ und $R^{10}$, die gleich oder verschieden sind, ein Wasserstoff-, Fluor-, Chlor- oder Bromatom, eine $(C_1-C_3)$-Alkylgruppe, eine $(C_1-C_2)$-Alkoxigruppe, eine Acetamino- oder eine $(C_1-C_2)$-Alkoxicarbonylgruppe oder eine Carboxylgruppe bedeuten,

$R^8$ ein Fluor-, Chlor-, oder Bromatom oder eine Methyl-, $(C_1-C_2)$-Alkoxi- oder Trifluormethylgruppe

X ein Sauerstoff- oder Schwefelatom und

n = 0, 1, 2, oder 3 bedeuten, wobei die Reste

$R^1$ und $R^2$ zusammen auch einen verzweigten oder unverzweigten Alkylenrest mit 3-6 C-Atomen oder den $(-CH_2CH_2OCH_2CH_2-)$-Rest und

$R^2$ und $R^3$ zusammen einen verzweigten oder unverzweigten Alkylenrest mit 2-8 C-Atomen, der zusätzlich durch eine $(C_2-C_4)$-Alkenylgruppe oder einen, gegebenenfalls mit einem Chlor- oder Bromatom oder einer Methyl-, Ethyl-, Methoxi- oder Ethoxigruppe substituierten, Phenylrest substituiert sein kann, oder den (-CH = CH-)-Rest bedeuten, und wobei im Fall n = 1, die Reste $R^7$ und $R^8$ zusammen auch eine Methylen- oder Ethylendioxygruppe bedeuten können, sowie deren physiologisch verträglichen säureadditions-Salzen, dadurch gekennzeichnet, daß man

a) ein Isocyanat der allgemeinen Formel IIa

$$R^2 - N = C = O \qquad \text{(IIa)}$$

in der $R^2$ außer Wasserstoff die in Anspruch 1 angegebenen Bedeutungen hat, mit Guanidin umsetzt und die dabei gebildete Reaktionsmischung anschließend weiter mit einer Verbindung der allgemeinen Formel III

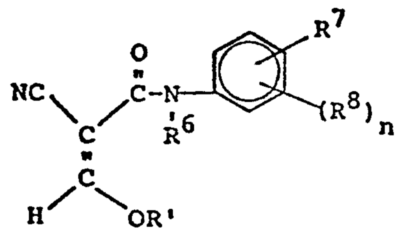

(III)

in der $R^6$, $R^7$ und $R^8$ sowie n die in Anspruch 1 bei der allgemeinen Formel I angegebenen Bedeutungen haben und R' eine Methyl- oder Ethylgruppe bedeutet, umsetzt oder

b) Verbindungen der allgemeinen Formel IV

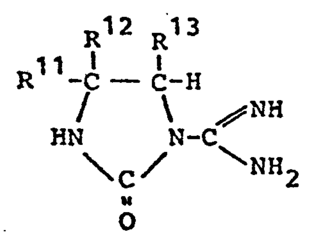

(IV)

in der $R^{11}$ Wasserstoff, $(C_1-C_3)$-Alkyl-, $(C_2-C_4)$-Alkenyl- oder, gegebenenfalls durch ein Chlor- oder Bromatom oder eine Methyl-, Ethyl, Methoxi- oder Ethoxigruppe substituiertes, Phenyl, und $R^{12}$ und $R^{13}$, die gleich oder verschieden sein können, jeweils Wasserstoff oder eine $(C_1-C_3)$-Alkylgruppe bedeuten, oder deren Säureadditionssalze und für den Fall, daß Salze von Verbindungen der allgemeinen Formel IV verwendet werden, unter Zusatz einer basischen Verbindung mit einer Verbindung der allgemeinen Formel III, in der $R^6$-$R^8$, R' und n die oben angegebene Bedeutung haben, unter Bildung einer Verbindung der allgemeinen Formel I, in der X = 0 bedeutet und $R^1$, $R^4$ und $R^5$ Wasserstoff, und $R^2$ und $R^3$ zusammen eine

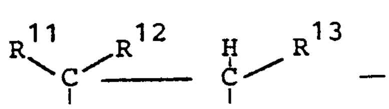

Gruppierung bedeuten, in der $R^{11}$-$R^{13}$ die obige Bedeutung haben, und $R^6$, $R^7$ und $R^8$ sowie n die oben genannte Bedeutung haben, umsetzt oder

c) ein Isocyanat der allgemeinen IIa, oder ein Isothiocyanat der allgemeinen Formel IIb

$R^2 — N = C = S$ (IIb)

in der $R^2$ außer Wasserstoff die in Anspruch 1 angegebene Bedeutung hat, mit einer Verbindung der allgemeinen Formel V

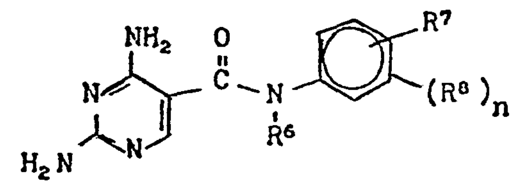

(V)

in der $R^6$, $R^7$ und $R^8$ sowie n die in Anspruch 1 bei der allgemeinen Formel I angegebenen Bedeutungen haben, umsetzt,

d) ein Chlorameinsäureamid bzw. ein Chlorthioameisensäureamid der allgemeinen Formel VI

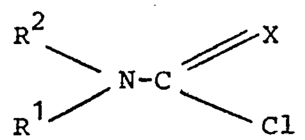

(VI)

in der $R^1$ eine $(C_1$-$C_3)$-Alkylgruppe oder einen Phenylrest, $R^2$ eine $(C_1$-$C_8)$-Alkylgruppe, eine $(C_3$-$C_4)$-Alkenylgruppe, eine $(C_5$-$C_6)$-Cycloalkylgruppe, eine Benzyl- oder Phenylgruppe, die gegebenenfalls im Phenylrest zusätzlich durch eine Methylgruppe, 1 oder 2 Chloratome, 1 oder 2 Methoxigruppen oder eine Methylendioxigruppe substituiert sein können, oder

$R^1$ und $R^2$ zusammen einen verzweigten oder unverzweigten Alkylenrest mit 3-6 C-Atomen oder den $(-CH_2CH_2OCH_2CH_2-)$-Rest und

X ein Sauerstoff- oder Schwefelatom bedeuten mit einer Verbindung der allgemeinen Formel V, in der $R^6$, $R^7$ und $R^8$ sowie n die in Anspruch 1 bei der allgemeinen Formel I angegebene Bedeutung haben, umsetzt, oder

e) 4-Amino-2-ureido-(bzw.-2-thioureido)-pyrimidin-5-carbonsäure-anilide der allgemeinen Formel I, in der $R^4$ Wasserstoff bedeutet und $R^1$, $R^2$, $R^3$, $R^5$, $R^6$, $R^7$, $R^8$, X und n die oben bei der allgemeinen Formel I angegebenen Bedeutungen haben, mit einem Acylierungsmittel der allgemeinen Formel VII

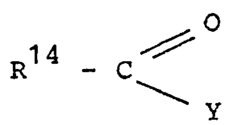

(VII)

in der

$R^{14}$ eine $(C_1$-$C_7)$-Alkylgruppe, einen Cyclohexyl- einen Benzyl- oder einen, gegebenenfalls durch eine Methylgruppe substituierten, Phenylrest und

Y ein Chlor- oder Bromatom oder die Gruppe $R^{14}—\overset{O}{\overset{\|}{C}}—O—$ bedeutet, umsetzt, und gegebenenfalls die nach Weg a) -e) erhaltenen Verbindungen der allgemeinen Formel I mit anorganischen oder organischen Säuren in ihre physiologisch verträglichen Säureadditionssalze überführt.

2. Verfahren gemäß Anspruch 1, dadurch gekennzeichnet, daß man 4-Amino-2-(4,4-dimethyl-imidazolidin-2-on-1-yl)-pyrimidin-5-carbonsäure-(3-trifluormethyl-anilid) bzw. physiologisch verträgliche Säureadditionssalze davon herstellt.

3. Verfahren gemäß Anspruch 1, dadurch gekennzeichnet, daß man 4-Amino-2-(4,4-dimethyl-imidazolidin-2-on-1-yl)-pyrimidin-5-carbonsäure-(3-chlor-2-methyl-anilid) bzw. physiologisch verträgliche Säureadditionssalze davon herstellt.

4. Verfahren gemäß Anspruch 1, dadurch gekennzeichnet, daß man 4-Amino-2-(4,4-dimethyl-imidazolidin-2-on-1-yl)-pyrimidin-5-carbonsäure-(2-fluor-5-trifluormethyl-anilid) bzw. physiologisch verträgliche Säureadditionssalze davon herstellt.

**Claims** (for the contracting States : BE, CH, DE, FR, GB, IT, NL, SE)

1. 4-Amino-2-ureido- (or -thioureido)-pyrimidine-5-carboxylic acid anilides of the general formula I

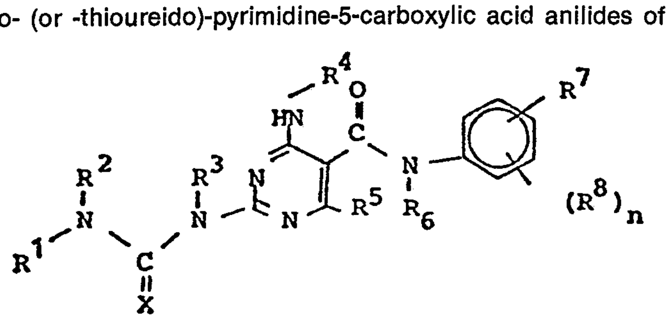

(I)

in which

$R^1$ and $R^3$, which are identical or different, denote hydrogen, a $(C_1$-$C_3)$-alkyl group or a phenyl radical,

$R^2$ denotes hydrogen, a $(C_1$-$C_{10})$-alkyl group, a $(C_3$-$C_4)$-alkenyl group, a $(C_4$-$C_8)$-cycloalkyl group, a benzyl group or a phenyl group, wherein the phenyl radicals additionally can be substituted by 1-2 $(C_1$-$C_3)$-alkyl groups, 1-2 halogen atoms, 1-2 $(C_1$-$C_4)$-alkoxy groups, a trifluoromethyl group, a methoxycarbonyl or ethoxycarbonyl group and/or a methylenedioxy group,

$R^4$ denotes hydrogen or a $(C_1$-$C_8)$-acyl group,

$R^5$ denotes hydrogen,

$R^6$ denotes hydrogen, a $(C_1$-$C_3)$-alkyl group, a $(C_3$-$C_4)$-alkenyl group, a benzyl group, which is optionally substituted in the phenyl radical by 1 or 2 chlorine atoms or 1 or 2 methoxy groups, or a phenyl radical, which is optionally substituted by 1 methyl group and/or 1 chlorine atom,

$R^7$ denotes hydrogen, a $(C_1$-$C_3)$-alkyl group, a $(C_3$-$C_4)$-alkenyl group, a $(C_5$-$C_6)$-cycloalkyl group, a phenyl group, a halogen atom, a trifluoromethyl, $(C_1$-$C_4)$-alkylthio, $(C_1$-$C_2)$-alkoxy-carbonyl, cyano, acetamino, amino, nitro, carboxyl or $(C_1$-$C_4)$-alkoxy group or a radical

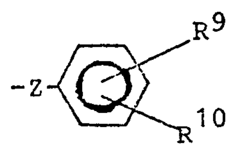

in which Z denotes an oxygen or sulfur atom or a $-\overset{O}{\overset{\|}{C}}-$, $-CH_2-$ or $-CH_2CH_2-$ group and $R^9$ and $R^{10}$, which are identical or different, denote a hydrogen, fluorine, chlorine or bromine atom, a $(C_1$-$C_3)$-alkyl group, a $(C_1$-$C_2)$-alkoxy group, an acetamino group or a $(C_1$-$C_2)$-alkoxy-carbonyl group or a carboxyl group, and

$R^8$ denotes a fluorine, chlorine or bromine atom or a methyl, $(C_1$-$C_2)$-alkoxy or trifluoromethyl group,

X denotes an oxygen or sulfur atom and

n denotes 0, 1, 2 or 3, and the radicals

$R^1$ and $R^2$ together also denote a branched or unbranched alkylene radical with 3-6 C atoms or denote the $(-CH_2CH_2OCH_2CH_2-)$ radical and

$R^2$ and $R^3$ together denote a branched or unbranched alkylene radical with 2-8 C atoms, which, in addition, can be susbstituted by a $(C_2$-$C_4)$-alkenyl group or a phenyl radical, which is optionally substituted by a chlorine or bromine atom or a methyl, ethyl, methoxy or ethoxy group, or denote the $(-CH = CH-)$ radical and, if n is 1, the radicals $R^7$ and $R^8$ together can also denote a methylenedioxy or ethylenedioxy group, and their physiologically acceptable acid addition salts.

2. 4-Amino-2-(4,4-dimethyl-imidazolidin-2-on-1-yl)-pyrimidine-5-carboxylic acid 3-trifluoromethyl-anilide according to claim 1.

3. 4-Amino-2-(4,4-dimethyl-imidazolidin-2-on-1-yl)-pyrimidine-5-carboxylic acid 3-chloro-2-methyl-anilide according to claim 1.

4. 4-Amino-2-(4,4-dimethyl-imidazolidin-2-on-1-yl)-pyrimidine-5-carboxylic acid 2-fluoro-5-trifluoromethyl-anilide according to claim 1.

5. Process for the preparation of compounds according to claim 1, which comprises

a) reacting an isocyanate of the general formula IIa

$$R^2 - N = C = O \qquad \text{(IIa)}$$

in which $R^2$ has the meanings indicated in claim 1 in addition to hydrogen, with guanidine and then further reacting the reaction mixture, thus formed, with a compound of the general formula III

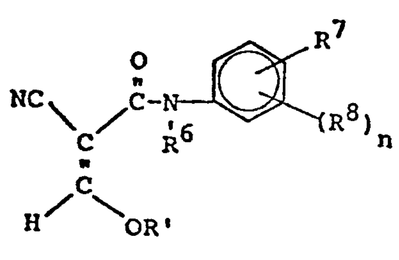

(III)

in which $R^8$, $R^7$ and $R^8$ and also n have the meanings indicated in claim 1 under the general formula 1 and R′ denotes a methyl or ethyl group, or

b) reacting compounds of the general formula IV

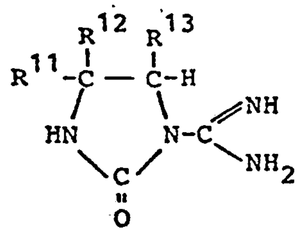

(IV)

in which $R^{11}$ denotes hydrogen, $(C_1$-$C_3)$-alkyl, $(C_2$-$C_4)$-alkenyl or phenyl, which is optionally substituted by a chlorine or bromine atom or a methyl, ethyl, methoxy or ethoxy group, and $R^{12}$ and $R^{13}$, which can be identical or different, each denote hydrogen or a $(C_1$-$C_3)$-alkyl group, or their acid addition salts, and, if salts of compounds of the general formula IV are used, with the addition of a basic compound, with a compound of the general formula III, in which $R^6$-$R^8$, R' and n have the meaning indicated above, with the formation of a coumpound of the general formula I in which X denotes 0 and $R^1$, $R^4$ and $R^5$ denote hydrogen and $R^2$ and $R^3$ together denote a

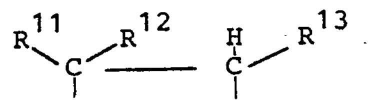

grouping, in which $R^{11}$-$R^{13}$ have the above meaning, and $R^6$ and $R^7$ and $R^8$ and also n have the meaning indicated above, or

c) reacting an isocyanate of the general formula IIa or an isothiocyanate of the general formula IIb

$$R^2 - N = C = S \quad \text{(IIb)}$$

in which $R^2$ has the meaning indicated in claim 1, in addition to hydrogen, with a compound of the general formula V

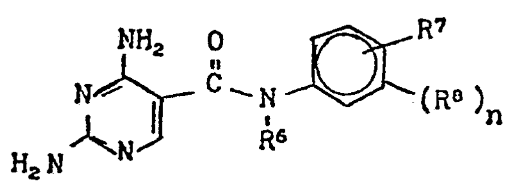

(V)

in which $R^6$, $R^7$ and $R^8$ and also n have the meanings indicated in claim 1 under the general formula I, or

d) reacting a chloroformic acid amide or a chlorothioformic acid amide of the general formula VI

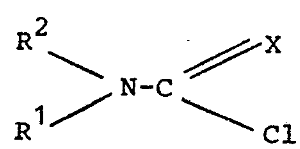

(VI)

in which $R^1$ denotes a $(C_1$-$C_3)$-alkyl group or a phenyl radical, $R^2$ denotes a $(C_1$-$C_8)$-alkyl group, a $(C_3$-$C_4)$-alkenyl group, a $(C_5$-$C_6)$-cycloalkyl group or a benzyl or phenyl group, which optionally can be additionally substituted in the phenyl radical by a methyl group, 1 or 2 chlorine atoms, 1 or 2 methoxy groups or a methylenedioxy group, or

$R^1$ and $R^2$ together denote a branched or unbranched alkylene radical with 3-6 C atoms or the $(-CH_2CH_2OCH_2CH_2-)$ radical, and

X denotes an oxygen or sulfur atom, with a compound of the general formula V in which $R^6$, $R^7$ and $R^8$ and also n have the meaning indicated in claim 1 under the general formula I, or

e) reacting 4-amino-2-ureido-(or-2-thioureido)-pyrimidine-5-carboxylic acid anilides of the general formula I, in which $R^4$ denotes hydrogen and $R^1$, $R^2$, $R^3$, $R^5$, $R^6$, $R^7$, $R^8$, X and n have the meanings indicated above under the general formula I, with an acylating agent of the general formula VII

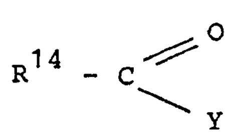

(VII)

in which

$R^{14}$ denotes a ($C_1$-$C_7$)-alkyl group, a cyclohexyl radical, a benzyl radical or a phenyl radical, which is optionally substituted by a methyl group, and

Y denotes a chlorine or bromine atom or the group $R^{14}—\overset{O}{\overset{\|}{C}}—O—$, and optionally converting the compounds of the general formula I obtained by route a) - e) to their physiologically acceptable acid addition salts using inorganic or organice acids.

6. Medicament based on a compound according to claim 1.

**Claims** (for the contracting State AT)

1. Process for the preparation of 4-amino-2-ureido-(or-thioureido)-pyrimidine-5-carboxylic acid anilides of the general formula I

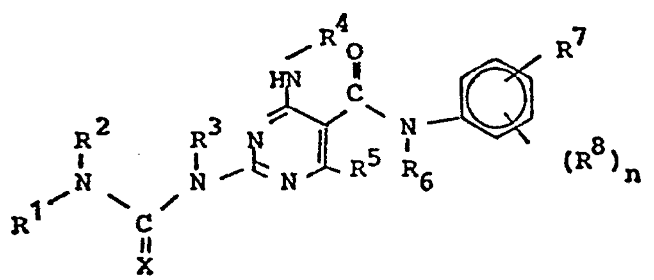

(I)

in which

$R^1$ and $R^3$, which are identical or different, denote hydrogen, a ($C_1$-$C_3$)-alkyl group or a phenyl radical,

$R^2$ denotes hydrogen, a ($C_1$-$C_{10}$)-alkyl group, a ($C_3$-$C_4$)-alkenyl group, a ($C_4$-$C_8$)-cycloalkyl group, a benzyl group or a phenyl group, wherein the phenyl radicals additionally can be substituted by 1-2 ($C_1$-$C_3$)-alkyl groups, 1-2-halogen atoms, 1-2 ($C_1$-$C_4$)-alkoxy groups, a trifluoromethyl group, a methoxycarbonyl or ethoxycarbonyl group and/or a methylenedioxy group,

$R^4$ denotes hydrogen or a ($C_1$-$C_8$)-acyl group,

$R^5$ denotes hydrogen,

$R^6$ denotes hydrogen, a ($C_1$-$C_3$)-alkyl group, a ($C_3$-$C_4$)-alkenyl group, a benzyl group, which is optionally substituted in the phenyl radical by 1 or 2 chlorine atoms or 1 or 2 methoxy groups, or a phenyl radical, which is optionally substituted by 1 methyl group and/or 1 chlorine atom,

$R^7$ denotes hydrogen, a ($C_1$-$C_3$)-alkyl group, a ($C_3$-$C_4$)-alkenyl group, a ($C_5$-$C_6$)-cycloalkyl group, a phenyl group, a halogen atom, a trifluoromethyl, ($C_1$-$C_4$)-alkylthio, ($C_1$-$C_2$)-alkoxy-carbonyl, cyano, acetamino, amino, nitro, carboxyl or ($C_1$-$C_4$)-alkoxy group or a radical

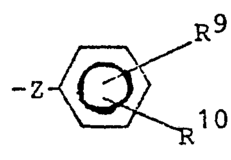

in which Z denotes an oxygen or sulfur atom or a -C-, $-CH_2-$ or $-CH_2CH_2-$ group and $R^9$ and $R^{10}$, which are identical or different, denote a hydrogen, fluorine, chlorine or bromine atom, a ($C_1$-$C_3$)-alkyl group, a ($C_1$-$C_2$)-alkoxy group, an acetamino group or a ($C_1$-$C_2$)-alkoxy-carbonyl group or a carboxyl group, and

$R^8$ denotes a fluorine, chlorine or bromine atom or a methyl, ($C_1$-$C_2$)-alkoxy or trifluoromethyl group,

X denotes an oxygen or sulfur atom and

n denotes 0, 1, 2 or 3, and the radicals

$R^1$ and $R^2$ together also denote a branched or unbranched alkylene radical with 3-6 C atoms or denote the ($-CH_2CH_2OCH_2CH_2-$) radical and

$R^2$ and $R^3$ together denote a branched or unbranched alkylene radical with 2-8 C atoms, which, in addition, can be substituted by a ($C_2$-$C_4$)-alkenyl group or a phenyl radical, which is optionally substituted by a chlorine or bromine atom or a methyl, ethyl, methoxy or ethoxy group, or denote the (-CH = CH-) radical and, if n is 1, the radicals $R^7$ and $R^8$ together can also denote a methylenedioxy or ethylenedioxy group, and of their physiologically acceptable acid addition salts, which comprises

a) reacting an isocyanate of the general formula IIa

$$R^2 - N = C = O \qquad \text{(IIa)}$$

in which $R^2$ has the meanings indicated in claim 1 in addition to hydrogen, with guanidine and then further reacting the reaction mixture, thus formed, with a compound of the general formula III

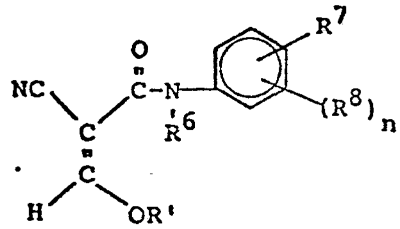

(III)

in which $R^6$, $R^7$ and $R^8$ and also n have the meanings indicated in claim 1 under the general formula 1 and R′ denotes a methyl or ethyl group, or

b) reacting compounds of the general formula IV

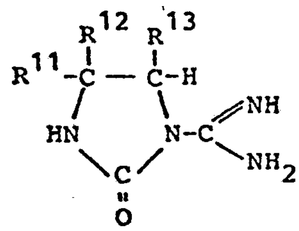

(IV)

in which $R^{11}$ denotes hydrogen, $(C_1\text{-}C_3)$-alkyl, $(C_2\text{-}C_4)$-alkenyl or phenyl, which is optionally substituted by a chlorine or bromine atom or a methyl, ethyl, methoxy or ethoxy group, and $R^{12}$ and $R^{13}$, which can be identical or different, each denote hydrogen or a $(C_1\text{-}C_3)$-alkyl group, or their acid addition salts, and, if salts of compounds of the general formula IV are used, with the addition of a basic compound, with a compound of the general formula III, in which $R^6$-$R^8$, R′ and n have the meaning indicated above, with the formation of a compound of the general formula I in which X denotes 0 and $R^1$, $R^4$ and $R^5$ denote hydrogen and $R^2$ and $R^3$ together denote a

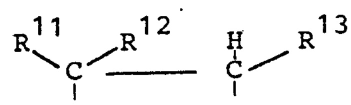

grouping, in which $R^{11}$-$R^{13}$ have the above meaning, and $R^6$ and $R^7$ and $R^8$ and also n have the meaning indicated above, or

c) reacting and isocyanate of the general formula IIa or an isothiocyanate of the general formula IIb

$$R^2 - N = C = S \qquad \text{(IIb)}$$

in which $R^2$ has the meaning indicated in claim 1, in addition to hydrogen, with a compound of the general formula V

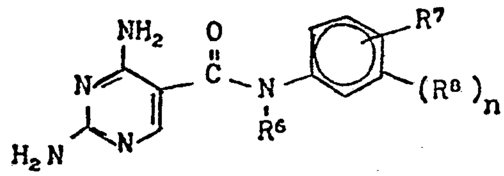

(V)

in which $R^6$, $R^7$ and $R^8$ and also n have the meanings indicated in claim 1 under the general formula I, or

d) reacting a chloroformic acid amide or a chlorothioformic acid amide of the general formula VI

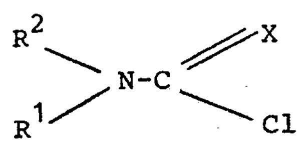

(VI)

in which $R^1$ denotes a ($C_1$-$C_3$)-alkyl group or a phenyl radical, $R^2$ denotes a ($C_1$-$C_8$)-alkyl group, a ($C_3$-$C_4$)-alkenyl group, a ($C_5$-$C_6$)-cycloalkyl group or a benzyl or phenyl group, which optionally can be additionally substituted in the phenyl radical by a methyl group, 1 or 2 chlorine atoms, 1 or 2 methoxy groups or a methylenedioxy group, or

$R^1$ and $R^2$ together denote a branched or unbranched alkylene radical with 3-6 C atoms or the ($-CH_2CH_2OCH_2CH_2-$) radical, and

X denotes an oxygen or sulfur atom, with a compound of the general formula V in which $R^6$, $R^7$ and $R^8$ and also n have the meaning indicated in claim 1 under the general formula I, or

e) reacting 4-amino-2-ureido-(or-2-thioureido)-pyrimidine-5-carboxylic acid anilides of the general formula I, in which $R^4$ denotes hydrogen and $R^1$, $R^2$, $R^3$, $R^5$, $R^6$, $R^7$, $R^8$, X and n have the meanings indicated above under the general formula I with an acylating agent of the general formula VII

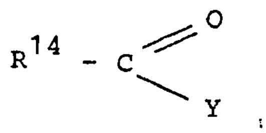

(VII)

in which

$R^{14}$ denotes a ($C_1$-$C_7$)-alkyl group, a cyclohexyl radical, a benzyl radical or a phenyl radical, which is optionally substituted by a methyl group, and

Y denotes a chlorine or bromine atom or the group $R^{14}-\overset{O}{\overset{\|}{C}}-O-$, and optionally converting the compounds of the general formula I obtained by route a) - e) to their physiologically acceptable acid addition salts using inorganic or organic acids.

2. Process according to claim 1, wherein the 4-amino-2-(4,4-dimethyl-imidazolidin-2-on-1-yl)-pyrimidine-5-carboxylic acid 3-trifluoromethyl-anilides or the physiologically acceptable acid addition salts thereof are prepared.

3. Process according to claim 1, wherein the 4-amino-2-(4,4-dimethyl-imidazolidin-2-on-1-yl)-pyrimidine-5-carboxylic acid 3-chloro-2-methyl-anilides or the physiologically acceptable acid addition salts thereof are prepared.

4. Process according to claim 1, wherein the 4-amino-2-(4,4-dimethyl-imidazolidin-2-on-1-yl)-pyrimidine-5-carboxylic acid 2-fluoro-5-trifluoromethyl-anilides or the physiologically acceptable acid addition salts thereof are prepared.

**Revendications** (pour les Etats contractants : BE, CH, DE, FR, GB, IT, NL, SE)

1. Anilides d'acides 4-amino-2-uréido-(ou -thiouréido)-pyrimidine-5-carboxyliques de formule générale I

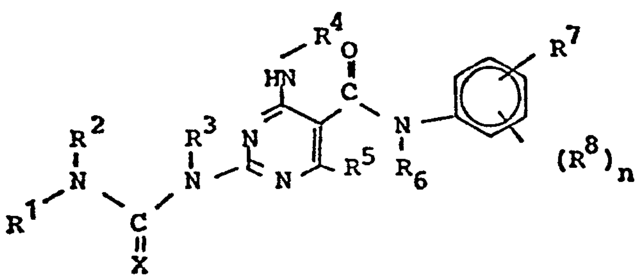

(I)

dans laquelle :

$R^1$ et $R^3$, qui sont identiques ou différents, représentent l'hydrogène, un groupe alkyle en $C_1$-$C_3$ ou un groupe phényle,

$R^2$ représente l'hydrogène, un groupe alkyle en $C_1$-$C_{10}$, un groupe alcényle en $C_3$-$C_4$, un groupe cycloalkyle en $C_4$-$C_8$, un groupe benzyle ou un groupe phényle dans lesquels les radicaux phényle peuvent en outre être substitués par 1 ou 2 groupes alkyle en $C_1$-$C_3$, 1 ou 2 atomes d'halogène, 1 ou 2 groupes alcoxy en $C_1$-$C_4$, un groupe trifluorométhyle, un groupe méthoxy- ou éthoxycarbonyle et/ou un groupe méthylènedioxy,

$R^4$ représente l'hydrogène ou un groupe acyle en $C_1$-$C_8$,

$R^5$ représente l'hydrogène

$R^6$ représente l'hydrogène, un groupe alkyle en $C_1$-$C_3$, un groupe alcényle en $C_3$-$C_4$, un groupe benzyle éventuellement substitué sur le radical phényle par 1 ou 2 atomes de chlore ou 1 ou 2 groupes

méthoxy, ou un groupe phényle éventuellement substitué par un groupe méthyle et/ou un atome de chlore,

$R^7$ représente l'hydrogène, un groupe alkyle en $C_1$-$C_3$, un groupe alcényle en $C_3$-$C_4$, un groupe cycloalkyle en $C_5$-$C_6$, un groupe phényle, un atome d'halogène, un groupe trifluorométhyle, alkylthio en $C_1$-$C_4$, alcoxy (en $C_1$-$C_2$) carbonyle, cyano, acétamino, amino, nitro, carboxyle ou alcoxy en $C_1$-$C_4$ ou un radical

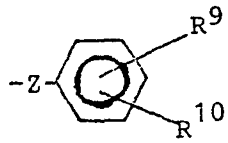

où Z représente un atome d'oxygène ou de soufre ou un groupe $-\overset{\overset{O}{\|}}{C}-$, $-CH_2-$ ou $-CH_2CH_2-$ et $R^9$ et $R^{10}$, qui sont identiques ou différents, représentent un atome d'hydrogène, de fluor, de chlore ou de brome, un groupe alkyle en $C_1$-$C_3$, un groupe alcoxy en $C_1$-$C_2$, un groupe acétamino ou alcoxy (en $C_1$-$C_2$) carbonyle ou un groupe carboxyle,

$R^8$ représente un atome de fluor, de chlore ou de brome ou un groupe méthyle, alcoxy en $C_1$-$C_2$ ou trifluorométhyle,

X représente un atome d'oxygène ou de soufre et

n = 0, 1, 2 ou 3, les radicaux

$R^1$ et $R^2$ représentant également ensemble un groupe alkylène ramifié ou non ramifié ayant de 3 à 6 atomes de carbone, ou le radical ($-CH_2CH_2OCH_2CH_2-$) et

$R^2$ et $R^3$ représentant ensemble un groupe alkylène ramifié ou non ramifié ayant de 2 à 8 atomes de carbone qui peut être en outre substitué par un groupe alcényle en $C_2$-$C_4$ ou un groupe phényle éventuellement substitué par un atome de chlore ou de brome ou par un groupe méthyle, éthyle, méthoxy ou éthoxy, ou le radical (-CH = CH-) — et dans le cas où n = 1, les radicaux $R^7$ et $R^8$ peuvent représenter également ensemble un groupe méthylène- ou éthylène-dioxy, ainsi que leurs sels d'addition avec des acides physiologiquement acceptables.

2. 3-Trifluorométhyl-anilide de l'acide 4-amino-2-(4,4-diméthyl-imidazolidine-2-one-1-yl)-pyrimidine-5-carboxylique selon la revendication 1.

3. 3-chloro-2-méthyl-anilide de l'acide 4-amino-2-(4,4-diméthyl-imidazolidine-2-one-1-yl)-pyrimidine-5-carboxylique selon la renvendication 1.

4. 2-fluoro-5-trifluorométhyl-anilide de l'acide 4-amino-2-(4,4-diméthyl-imidazolidine-2-one-1-yl)-pyrimidine-5-carboxylique selon la revendication 1.

5. Procédé de préparation des composés selon la revendication 1, caractérisé en ce que :

a) on fait réagir un isocyanate de formule générale IIa

$$R^2 — N = C = O \qquad \text{(IIa)}$$

où $R^2$, en dehors de l'hydrogène, a la signification indiquée dans la revendication 1, avec de la guanidine, puis on fait réagir le mélange réactionnel ainsi formé avec un composé de formule générale III

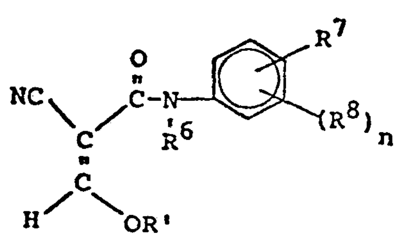

(III)

où $R^6$, $R^7$ et $R^8$ ainsi que n ont les significations indiquées dans la revendication 1 pour la formule générale I et R' représente un groupe méthyle ou éthyle, ou

b) on fait réagir des composés de formule générale IV

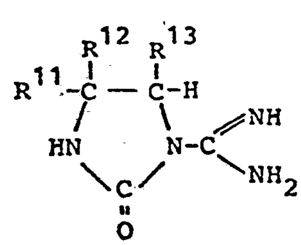

(IV)

où $R^{11}$ représente l'hydrogène, un groupe alkyle en $C_1$-$C_3$, un groupe alcényle en $C_2$-$C_4$ ou un groupe phényle éventuellement substitué par un atome de chlore ou de brome ou par un groupe méthyle, éthyle, méthoxy ou éthoxy, et $R^{12}$ et $R^{13}$, qui peuvent être identiques ou différents, représentent chacun l'hydrogène ou un groupe alkyle en $C_1$-$C_3$, ou leurs sels d'addition avec des acides et, dans le cas où on utilise des sels des composés de formule générale IV, en présence d'un composé basique, avec un composé de formule générale III où $R^6$-$R^8$, R' et n ont les significations indiquées ci-dessus, pour former un composé de formule générale I où X = 0 et $R^1$, $R^4$ et $R^5$ représentent l'hydrogène et $R^2$ et $R^3$ représentent ensemble un groupe

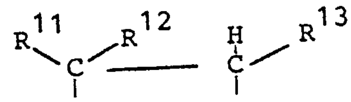

où $R^{11}$-$R^{13}$ ont les significations indiquées ci-dessus, et $R^6$, $R^7$ et $R^8$ ainsi que n ont les significations indiquées ci-dessus, ou

c) on fait réagir un isocyanate de formule générale IIa ou un isothiocyanate de formule générale IIb

$$R^2 - N = C = S \qquad \text{(IIb)}$$

où $R^2$, en dehors de l'hydrogène, a la signification indiquée dans la revendication 1, avec un composé de formule générale V

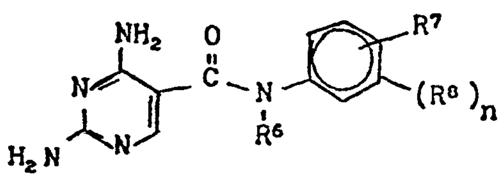

(V)

dans laquelle $R^6$, $R^7$ et $R^8$ ainsi que n ont les significations indiquées dans la revendication 1 pour la formule générale I, ou

d) on fait réagir un amide de l'acide chloroformique ou un amide de l'acide chlorothioformique de formule générale VI

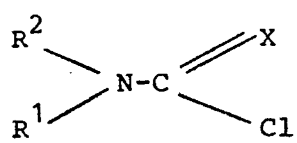

(VI)

dans laquelle $R^1$ représente un groupe alkyle en $C_1$-$C_3$ ou un groupe phényle, $R^2$ représente un groupe alkyle en $C_1$-$C_8$, un groupe alcényle en $C_3$-$C_4$, un groupe cycloalkyle en $C_5$-$C_6$, un groupe benzyle ou phényle qui peuvent être en outre éventuellement substitués sur le radical phényle par un groupe méthyle, 1 ou 2 atomes de chlore, 1 ou 2 groupes méthoxy ou un groupe méthylènedioxy, ou

$R^1$ et $R^2$ ensemble représentent un groupe alkylène ramifié ou non ramifié ayant de 3 à 6 atomes de carbone ou un radical ($-CH_2CH_2OCH_2CH_2-$) et

X représente un atome d'oxygène ou de soufre, avec un composé de formule générale V où $R^6$, $R^7$ et $R^8$ ainsi que n ont les significations indiquées dans la revendication 1 pour la formule générale I, ou

e) on fait réagir des anilides d'acides 4-amino-2-uréido- ou (-2-thiouréido)-pyrimidine-5-carboxyliques de formule générale I, dans laquelle $R^4$ représente l'hydrogène et $R^1$, $R^2$, $R^3$, $R^5$, $R^6$, $R^7$, $R^8$, X et n ont les significations indiquées ci-dessus pour la formule générale I, avec un agent d'acylation de formule générale VII

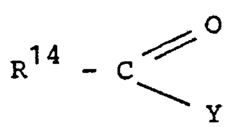

(VII)

dans laquelle

$R^{14}$ représente un groupe alkyle en $C_1$-$C_7$, un groupe cyclohexyle, benzyle ou un groupe phényle éventuellement substitué par un groupe méthyle, et

Y représente un atome de chlore ou de brome ou le groupe $R^{14}\text{—}\overset{O}{\overset{\|}{C}}\text{—}O$, et éventuellement on convertit les composés de formule générale I obtenus selon les modes opératoires a) à e), avec des acides

minéraux ou organiques, en leurs sels d'addition avec des acides physiologiquement acceptables.

6. Médicament contenant un composé selon la revendication 1.

**Revendications** (pour l'Etat contractant AT)

1. Procédé de préparation d'anilides d'acides 4-amino-2-uréido- (ou -thiouréido)-pyrimidine-5-carboxyliques de formule générale I

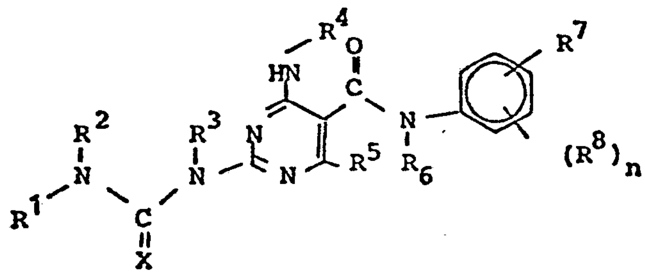

(I)

dans laquelle :

$R^1$ et $R^3$, qui sont identiques ou différents, représentent l'hydrogène, un groupe alkyle en $C_1$-$C_3$ ou un groupe phényle,

$R^2$ représente l'hydrogène, un groupe alkyle en $C_1$-$C_{10}$, un groupe alcényle en $C_3$-$C_4$, un groupe cycloalkyle en $C_4$-$C_8$, un groupe benzyle ou un groupe phényle dans lesquels les radicaux phényle peuvent en outre être substitués par 1 ou 2 groupes alkyle en $C_1$-$C_3$, 1 ou 2 atomes d'halogène, 1 ou 2 groupes alcoxy en $C_1$-$C_4$, un groupe trifluorométhyle, un groupe méthoxy- ou éthoxy-carbonyle et/ou un groupe méthylènedioxy,

$R^4$ représente l'hydrogène ou un groupe acyle en $C_1$-$C_8$,

$R^5$ représente l'hydrogène,

$R^6$ représente l'hydrogène, un groupe alkyle en $C_1$-$C_3$, un groupe alcényle en $C_3$-$C_4$, un groupe benzyle éventuellement substitué sur le radical phényle par 1 ou 2 atomes de chlore ou 1 ou 2 groupes méthoxy, ou un groupe phényle éventuellement substitué par un groupe méthyle et/ou un atome de chlore,

$R^7$ représente l'hydrogène, un groupe alkyle en $C_1$-$C_3$, un groupe alcényle en $C_3$-$C_4$, un groupe cycloalkyle en $C_5$-$C_6$, un groupe phényle, un atome d'halogène, un groupe trifluorométhyle, alkylthio en $C_1$-$C_4$, alcoxy (en $C_1$-$C_2$) carbonyle, cyano, acétamino, amino, nitro, carboxyle ou alcoxy en $C_1$-$C_4$ ou un radical

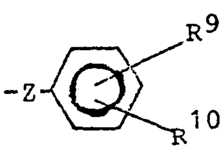

où Z représente un atome d'oxygène ou de soufre ou un groupe $-\overset{O}{\overset{\|}{C}}-$, $-CH_2-$ ou $-CH_2CH_2-$ et $R^9$ et $R^{10}$, qui sont identiques ou différents, représentent un atome d'hydrogène, de fluor, de chlore ou de brome, un groupe alkyle en $C_1$-$C_3$, un groupe alcoxy en $C_1$-$C_2$, un groupe acétamino ou alcoxy (en $C_1$-$C_2$) carbonyle ou un groupe carboxyle,

$R^8$ représente un atome de fluor, de chlore ou de brome ou un groupe méthyle, alcoxy en $C_1$-$C_2$ ou trifluorométhyle,

X représente un atome d'oxygène ou de soufre et

n = 0, 1, 2 ou 3, les radicaux

$R^1$ et $R^2$ représentant également ensemble un groupe alkylène ramifié ou non ramifié ayant de 3 à 6 atomes de carbone ou le radical $(-CH_2CH_2OCH_2CH_2-)$ et

$R^2$ et $R^3$ représentant ensemble un groupe alkylène ramifié ou non ramifié ayant de 2 à 8 atomes de carbone qui peut être en outre substitué par un groupe alcényle en $C_2$-$C_4$ ou un groupe phényle éventuellement substitué par un atome de chlore ou de brome ou par un groupe méthyle, éthyle, méthoxy ou éthoxy, ou le radical (-CH = CH-) et dans le cas où n = 1, les radicaux $R^7$ et $R^8$ peuvent représenter également ensemble un groupe méthylène- ou éthylène-dioxy, ainsi que leurs sels d'addition avec des acides physiologiquement acceptables, caractérisé en ce que :

a) on fait réagir un isocyanate de formule générale IIa

$$R^2 - N = C = O \qquad \text{(IIa)}$$

où $R^2$, en dehors de l'hydrogène, a la signification indiquée dans la revendication 1, avec de la guanidine, puis on fait réagir le mélange réactionnel ainsi formé avec un composé de formule générale III

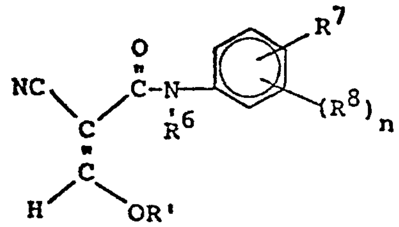
(III)

où $R^6$, $R^7$ et $R^8$ ainsi que n ont les significations indiquées dans la revendication 1 pour la formule générale I et R' représente un groupe de méthyle ou éthyle, ou

b) on fait réagir des composés de formule générale IV

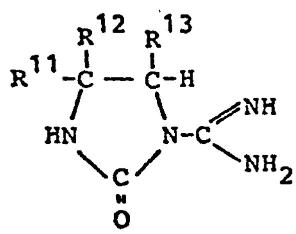
(IV)

où $R^{11}$ représente l'hydrogène, un groupe alkyle en $C_1$-$C_3$, un groupe alcényle en $C_2$-$C_4$ ou un groupe phényle éventuellement substitué par un atome de chlore ou de brome ou par un groupe méthyle, éthyle, méthoxy ou éthoxy, et $R^{12}$ et $R^{13}$, qui peuvent être identiques ou différents, représentent chacun l'hydrogène ou un groupe alkyle en $C_1$-$C_3$, ou leurs sels d'addition avec des acides et, dans le cas où on utilise des sels des composés de formule générale IV, en présence d'un composé basique, avec un composé de formule générale III où $R^6$-$R^8$, R' et n ont les significations indiquées ci-dessus, pour former un composé de formule générale I où X = 0 et $R^1$, $R^4$ et $R^5$ représentent l'hydrogène et $R^2$ et $R^3$ représentent ensemble un groupe

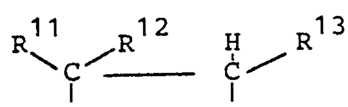

où $R^{11}$-$R^{13}$ ont les significations indiquées ci-dessus et $R^6$, $R^7$ et $R^8$ ainsi que n ont les significations indiquées ci-dessus, ou

c) on fait réagir un isocyanate de formule générale IIa ou un isothiocyanate de formule générale IIb

$$R^2 - N = C = S \qquad \text{(IIb)}$$

où $R^2$, en dehors de l'hydrogène, a la signification indiquée dans la revendication 1, avec un composé de formule générale V

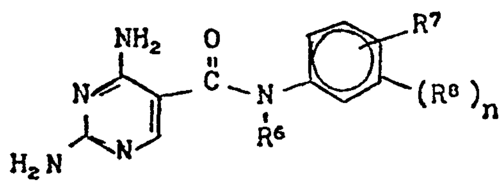
(V)

dans laquelle $R^6$, $R^7$ et $R^8$ ainsi que n ont les significations indiquées dans la revendication 1 pour la formule générale I, ou

d) on fait réagir un amide de l'acide chloroformique ou un amide de l'acide chlorothioformique de formule générale VI

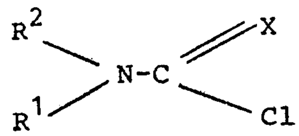

(VI)

dans laquelle $R^1$ représente un groupe alkyle en $C_1$-$C_3$ ou un groupe phényle, $R^2$ représente un groupe alkyle en $C_1$-$C_8$, un groupe alcényle en $C_3$-$C_4$, un groupe cycloalkyle en $C_5$-$C_6$, un groupe benzyle ou phényle qui peuvent être en outre éventuellement substitués sur le radical phényle par un groupe méthyle, 1 ou 2 atomes de chlore, 1 ou 2 groupes méthoxy ou un groupe méthylènedioxy, ou

$R^1$ et $R^2$ ensemble représentent un groupe alkylène ramifié ou non ramifié ayant de 3 à 6 atomes de carbone ou un radical ($-CH_2CH_2OCH_2CH_2-$) et

X représente un atome d'oxygène ou de soufre, avec un composé de formule générale V où $R^6$, $R^7$ et $R^8$ ainsi que n ont les significations indiquées dans la revendication 1 pour la formule générale I, ou

e) on fait réagir des anilides d'acides 4-amino-2-uréido- (ou-2-thiouréido)-pyrimidine-5-carboxyliques de formule générale I, dans laquelle $R^4$ représente l'hydrogène et $R^1$, $R^2$, $R^3$, $R^5$, $R^6$, $R^7$, $R^8$, X et n ont les significations indiquées ci-dessus pour la formule générale I, avec un agent d'acylation de formule générale VII

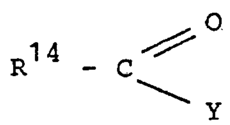

(VII)

dans laquelle :

$R^{14}$ représente un groupe alkyle en $C_1$-$C_7$, un groupe cyclohexyle, benzyle ou un groupe phényle éventuellement substitué par un groupe méthyle, et

Y représente un atome de chlore ou de brome ou le groupe $R^{14}-\overset{\overset{O}{\|}}{C}-O$, et éventuellement on convertit les composés de formule générale I obtenus selon les modes opératoires a) à e), avec des acides minéraux ou organiques, en leurs sels d'addition avec des acides physiologiquement acceptables.

2. Procédé selon la revendication 1, caractérisé en ce qu'on prépare le 3-trifluorométhyl-anilide de l'acide 4-amino-2-(4,4-diméthyl-imidazolidine-2-one-1-yl)-pyrimidine-5-carboxylique ou ses sels d'addition avec des acides physiologiquement acceptables.

3. Procédé selon la revendication 1, caractérisé en ce qu'on prépare le 3-chloro-2-méthyl-anilide de l'acide 4-amino-2-(4,4-diméthyl-imidazolidine-2-one-1-yl)-pyrimidine-5-carboxylique ou ses sels d'addition avec des acides physiologiquement acceptables.

4. Procédé selon la revendication 1, caractérisé en ce qu'on prépare le 2-fluoro-5-trifluorométhyl-anilide de l'acide 4-amino-2-(4,4-diméthyl-imidazolidine-2-one-1-yl)-pyrimidine-5-carboxylique ou ses sels d'addition avec des acides physiologiquement acceptables.